# EUROPEAN PATENT SPECIFICATION

(11) **EP 3 925 551 B1**
(45) Date of publication and mention of the grant of the patent: **10.07.2024**
(21) Application number: 21179974.7
(22) Date of filing: 17.06.2021
(51) Int. Cl.: A61B 17/221, A61B 17/22

(54) **CATHETER MOUTH DESIGNS**
KATHETERMÜNDUNGSENTWÜRFE
CONCEPTIONS DE BOUCHE DE CATHÉTER

(30) Priority: 18.06.2020 US 202016946371
(43) Date of publication of application: 22.12.2021
(73) Proprietor: Neuravi Limited, Galway H91 K5YD (IE)
(72) Inventor: VALE, David, Galway, H91 K5YD (IE); KEATING, Karl, Galway, H91 K5YD (IE); KELLY, Ronald, Galway, H91 K5YD (IE)
(74) Representative: Carpmaels & Ransford LLP

(56) References cited:
- EP-A1- 3 420 978
- WO-A1-2005/027751
- WO-A1-2019/064306
- WO-A1-2019/079296
- US-A1- 2011 125 181
- US-A1- 2016 228 134
- US-A1- 2017 105 743
- US-A1- 2019 239 907
- US-A1- 2019 365 411

## Description

### Field of the Invention

The present disclosure generally relates to devices and not claimed methods for removing acute blockages from blood vessels during intravascular medical treatments. More specifically, the present disclosure relates to an expandable tip for a retrieval aspiration catheter.

### Background

Aspiration and clot retrieval catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and are highly tortuous.

In delivering effective devices to the small and highly branched cerebral artery system, conventional catheters must try and balance a number of factors. The catheter must be sufficiently flexible to navigate the vasculature and endure high flexure strains, while also having the axial stiffness to offer smooth and consistent advancement along the route. Additionally, abrupt stiffness or geometric changes can hinder trackability, introduce significant stress concentrations, and increase the likelihood of device kinking or buckling.

Some designs for aspirating clot retrieval catheters, such as those with fixed mouths, can have difficulty directing the full suction of aspiration to the volume of fluid and clot distal to the mouth. When aspirating with a catheter which is incapable of sealing with the target vessel, a significant portion of the aspiration flow ends up coming from vessel fluid proximal to the catheter tip, as opposed to the distal vessel regions with the clot. This significantly reduces aspiration efficiency and lowers the success rate of clot removal. Furthermore firm, fibrin-rich clots can often be difficult to extract as they can become lodged in the tip of traditional fixed-mouth catheters. This lodging can cause softer portions to shear away from the firmer regions of the clot.

Designs for aspirating catheters which feature a larger or expandable mouth for improved efficiency must balance the flexibility for delivery with adequate radial force and atraumatic deployment. Catheter elements must survive the severe mechanical strains imparted but also generate a sufficient radial force when expanded to prevent collapse under the suction of aspiration. To meet these requirements, the mouth of some aspiration catheters have been designed with diameters that are considerably larger than the typical delivery catheter or sheath. These designs can fail to effectively balance the competing requirements to truly be effective and safe for a wide variety of procedural conditions

The present designs are aimed at providing an improved retrieval catheter with an expansile tip which incorporates features to address the above-stated deficiencies.

US 2011/125181 A1 relates to a clot capture system for disengaging a clot from a vessel wall and removing the clot from the vessel, that comprises a clot capture device for placement on a distal side of a clot. The clot capture device has a retracted delivery configuration and an expanded deployed configuration. The clot removal device has a proximal support frame, and a distal fibre net. The support frame has a retracted delivery configuration and an expanded deployed configuration.

US 2019/365411 A1 relates to a medical device for minimally invasive removal of tissue from a body lumen, that can include: a sheath having a proximal and a distal end; and a tool configured to pass through the sheath and configured to transition from a crimped state to a deployed state and to a closed state, wherein the tool forms an aperture at a distal end, the distal end of the tool having at least one tube loop and at least one wire loop, wherein the tool is configured to dissect tissue in the deployed state.

WO 2005/027751 A1 relates to a thrombus/embolus collecting device, comprising a body part freely radially contracted and enlarged and normally radially enlarged in a cylindrical shape, a tail part formed continuously with the body part, freely radially contracted and enlarged, and normally radially enlarged in a tapered shape, a stent formed continuously with the tail part and formed of a strut part, and a bag-like (conical- shaped) filter formed of an opening part and a body part.

WO 2019/079296 A1 relates to a device comprising a generally cylindrical stent body, and a distal net. The stent body is configured to engage and capture emboli, while the distal net is configured to restrict blood flow, and capture embolic debris.

US 2017/105743 A1 relates to a catheter that has an expansile tip that can be delivered in a constricted form. The increased tip diameter facilitates the aspiration and removal of large clot volumes by increasing the area of the catheter tip that applies aspiration to the clot. The tip comprises a support frame which can be cut from a metal or polymer tube, covered with a thin polymer sleeve containing highly elastic properties.

US 2016/228134 A1 relates to structures for removing obstructions from body lumens.

WO 2019/064306 A1 relates to an elongated braid structure configured for transitioning between collapsed and expanded states, the elongated braid structure surrounding a lumen having a distal opening when in the expanded state.

EP 3420978 A1 relates to a clot removal device for removing clots from a body vessel comprising an expandable structure and an elongate member. The elongate member can have a proximal end and a distal end, the elongate member being connected to the expandable structure at its distal end.

US 2019/239907 A1 relates to A method of removing clot from a vessel, including delivering a clot retrieval device across the clot, wherein the clot retrieval device includes an inner body having a collapsed delivery configuration and an expanded deployed configuration; and an outer body extending along a longitudinal axis and at least partially overlying the inner body.

### Summary

The invention is defined in claim 1. Embodiments of the invention are defined in the dependent claims. The designs herein can be for an expandable distal tip of a clot retrieval catheter capable of providing local flow restriction/arrest within the target vessel with a large clot-facing mouth. The catheter can be sufficiently flexible so as to be capable of navigating highly tortuous areas of the anatomy, such as the neurovascular, to reach an occlusive clot. The expandable tip of the catheter can also be compatible with relatively low-profile access sheaths and catheters for deliverability advantages.

The clot retrieval catheter can have a substantially tubular support tube defining a longitudinal axis. A large central catheter lumen can be configured for the passage of guidewires, microcatheters, stent retrievers, and other such devices. The lumen can also direct aspiration to the catheter tip. The tubular body can terminate at a distal end, at which an expansile tip can be integrally formed or fixedly connected.

The catheter can have a self-expanding mouth framework with a plurality of interconnected struts formed into a porous framework. The mouth framework can be configured to expand from a collapsed delivery configuration to an expanded deployed configuration when deployed at the site of an occlusive thrombus. In the expanded deployed configuration, the tip can assume a substantially conical or funnel shape. The funnel shape formed by the tip can improve aspiration efficiency, arrest unwanted flow, and lessen the risk of vessel trauma from snagging on vessel openings.

In the deployed state the expansile tip is tapered such that a proximal end of the tip has a first radial dimension and a more distal portion of the tip has a second radial dimension larger than the first radial dimension. The second radial dimension can be larger than the diameter of the target blood vessel. At least a portion of the tip can have a radial dimension in the expanded deployed configuration greater than an inner diameter of an outer catheter.

In another example, at least a portion of the struts forming the perimeter of the mouth framework can extend radially inward in a distal direction from the peak maximum radial size of the mouth framework, such that the maximum radial size occurs at an axial location intermediate the proximal end and the distal end of the framework. Such a configuration can allow the tip to contact vessel walls in the expanded state with a large and gentle radius so as to avoid vessel trauma and reduce friction. When expanded and unconstrained, the diameter of the tip framework can range from 1 mm to 10 mm, and preferably closer to 3 mm.

The strut framework of the mouth can be a cut pattern of sheet or tube stainless steel, or a superelastic shape memory alloy such as Nitinol. The struts of the mouth framework can connect to form closed cells, loops, or undulating patterns. A plurality of distal hoops or crown struts can form the circumferential perimeter of the tip mouth opening. One or more support arm struts can extend longitudinally between the proximal and distal ends of the mouth framework to link adjacent hoops where they meet at hoop troughs, and the support arms can extend proximally from the hoop troughs to connect the expansile tip with the support tube and form a substantially conical surface about the longitudinal axis.

The catheter body can feature a combination of ribs and spines to define a substantially tubular shape. The expandable mouth can be formed integrally with the support tube for a monolithic structure, such as through machining the tube and mouth together from the same hypotube stock. In another example the tubular body can be of a metallic or polymeric braid/mesh or of coiled wire construction.

The support arms may be axisymmetric with the longitudinal axis of the catheter, or they can be twisted or situated in a helical fashion about the axis. Individual support arms can attach independently to a most distal rib or can extend from or align with one of the one or more axial spines of the support tube. As an alternative, some of the support arms can connect via slots, eyelets, or some other non-rigid connection such that the arms do not add stiffness to the strut framework.

The struts of the hoops and support arms can also contain features such as narrowed segments, curves, and/or undulations to enhance or tailor the flexibility of the structure. The support arms can take a waveform or sinusoidal pattern circumferentially to allow more freedom of bending along the axis of the arms. In another case, the struts of support arms can have a portion which is narrower in width than another portion of the support arms, or the support arms can have a width different than the width of at least part of the distal hoops or crowns making up the mouth perimeter.

The struts of the hoops and support arms of the mouth framework can intersect at multiple troughs located at various axial and clocking positions around the longitudinal axis. The number and location of trough intersection points can, in part, help determine the localized stiffness of the framework. For example, a support arm can terminate proximally at a support trough and distally at a hoop trough to form a closed cell. In one case, adjacent support arms can share one or more cells. In another example, support arms can extend proximally from an intersection with one or more hoops at a hoop trough and terminate at a spine, or at the most distal rib, of the support tube to form closed cells. These cells can help the mouth framework to elongate or shorten longitudinally under tensile or compressive loads during the thrombectomy procedure.

When the tip is in the collapsed delivery configuration the troughs of the framework can serve as hinges about which the strut framework folds. When expanded, the support arms of the mouth framework can form an angle with the longitudinal axis, the angle determining the rate of taper for the conical funnel shape of the expanded tip. The angle can, for example, have a range from approximately 10 degrees to approximately 45 degrees. In another example, the taper can shallow and the angle between the support arms and the longitudinal axis can be approximately 30 degrees.

The strut framework can be a cut pattern of sheet or tube stainless steel, or a superelastic shape memory alloy such as Nitinol. The funnel shape formed by the tip can improve aspiration efficiency, reduce friction, and lessen the risk of vessel trauma from snagging on vessel openings. A funnel shape also means in the deployed state the expansile tip is tapered such that a proximal end of the tip has a first radial dimension and a more distal portion of the tip has a second radial dimension larger than the first radial dimension. The second radial dimension can be larger than the diameter of the target blood vessel.

The catheter can further have a flexible elastomeric cover disposed radially so that if forms a sleeve around the at least part of the support tube and expandable tip of the clot retrieval catheter. The cover can be homogenous or can have multiple layers. As an alternative, the cover can be one or more polymer jackets.

Another expandable mouth for a clot retrieval catheter can have a self-expanding mouth framework disposed around a longitudinal axis. The mouth framework can have a collapsed delivery configuration when being delivered to a target site constrained within an outer catheter, and an expanded deployed configuration when the outer catheter is retracted to uncover the framework. The mouth framework can have a plurality of interconnected struts, and the struts can form petal-like shapes disposed circumferentially around the longitudinal axis. Each petal can have longitudinal arm struts behaving in a similar fashion to the support arms previously described. The petals can have undulations or have variable width with narrowed segments for enhanced flexibility. These features can encourage bending and flexing along axis of each arm. The longitudinal arms can extend individually, or one or more longitudinal arm struts can split to form one or more closed cells linked by distal hoops. The cells can allow the petals to elongate independently so as to avoid having the support tube pull the mouth framework proximally during clot retraction.

A polymeric membrane or cover can be disposed over, around, or encapsulating the mouth framework so that support is given to the membrane when the suction force of aspiration is directed through the catheter during a thrombectomy procedure. The cover can be taut so that it expands under the radial force of the mouth framework when the tip expands to the deployed configuration, or it can be loose or baggy so all the radial force can be directed to the vessel walls.

The petals of the self-expanding mouth framework can be made more flexibly by connecting the longitudinal support arms proximally at troughs, connecting struts, common spines, or individually to a most distal rib of the catheter support tube. The distal peak of each petal can be a crown or hoop member which is not connected circumferentially to adjacent petal crowns, so that each petal is independently sprung from its proximal connection or connections. As a result, petals can react to forces and clot morphologies separately so that each petal can flex individually without the constraint of adjacent petals.

In a further example, an expandable mouth for a clot retrieval catheter can have a proximal end, a distal end, and a radial strand array forming a closed cell mesh disposed around a longitudinal axis and extending from the proximal end to the distal end. The mesh array can be made of wire or shape memory alloy such that the mouth can expand from a collapsed delivery configuration to an enlarged deployed configuration. When unconstrained by an outer catheter in the enlarged deployed configuration, the cell mesh can form a substantially conical surface about the longitudinal axis. Similar to other examples, a flexible polymeric membrane can cover some or all of the closed cell mesh of the catheter tip.

The closed cell mesh array of the mouth framework can be a continuous polygonal pattern such as triangular or quadrilateral cells which are interlocked through the vertices of the adjacent cells. The pattern can be one of those commonly seen in stenting applications, where a minimally invasive mesh is used to support and hold open vessel passages. In one case, an elongated quadrilateral pattern forms cells pores where local array peaks mark the shared vertices. The pattern can repeat in an axial and radial fashion and the distalmost array peaks of adjacent pores can be joined by curved distal hoops or crowns to mark the perimeter of the expandable mouth.

The density of the closed cell mesh pattern can vary. A denser mesh can have a greater stiffness and radial force, but also provide more support for an overlaying cover or membrane. In one example, the pattern can be sufficiently dense to where blood flow across the mesh is impeded by the small size of the pores. In this situation a membrane cover may not be necessary as the pattern of the pores is fine enough to function as seal to block off blood within the vessel which is proximal of the tip.

In order to allow for smooth delivery of the clot retrieval catheter through an outer catheter, the closed cell mesh of the tip and/or the outer surface of the membrane or outer jackets can be coated with a low-friction, such as PTFE or FEP, or a or hydrophilic lubricious material such as those offered by Surmodics, DSM, and Harland medical. The coating can prevent a buildup of static or dynamic friction, mitigating the risk of the catheter binding or kinking in tortuous areas of the vasculature.

Other aspects and features of the present disclosure will become apparent to those of ordinary skill in the art, upon reviewing the following detailed description in conjunction with the accompanying figures.

### Brief Description of the Drawings

The above and further aspects of this invention are further discussed with reference to the following description in conjunction with the accompanying drawings, where like reference numbers indicate elements which are functionally similar or identical. The drawings are not necessarily to scale, emphasis instead being placed upon illustrating principles of the invention. The figures depict one or more implementations of the inventive devices, by way of example only, not by way of limitation.
Fig. 1 is an isometric view of a catheter support tube and expandable distal tip according to aspects of the present invention;
Fig. 2 shows the expanded distal tip of the catheter in the process of retrieving a clot from the target site in a vessel according to aspects of the present invention;
Figs. 3a-d illustrate isometric and profile views an example expandable tip according to aspects of the present invention;
Figs. 4a-d show views of another expandable tip according to aspects of the present invention;
Figs. 5a-d are views of another example expandable tip according to aspects of the present invention;
Figs. 6a-d are views of alternative expandable tip according to aspects of the present invention;
Figs. 7a-d are views of another expandable tip design according to aspects of the present invention;
Figs. 8a-d show a further example expandable tip according to aspects of the present invention;
Figs. 9a-d are views of an alternative expandable tip according to aspects of the present invention;
Figs. 10a-d illustrates another expandable tip according to aspects of the present invention;
Figs. 11a-d are various views of another expandable tip design according to aspects of the present invention;
Figs. 12a-d are views of a further expandable tip according to aspects of the present invention;
Figs. 13a-d are views of another expandable tip according to aspects of the present invention;
Figs. 14a-d illustrates an alternative expandable tip according to aspects of the present invention;
Figs. 15a-d show views of another design for an expandable tip according to aspects of the present invention;
Figs. 16a-d are views of another example expandable tip according to aspects of the present invention;
Figs. 17a-d are views of alternative expandable tip according to aspects of the present invention;
Figs. 18a-d show views of another expandable tip according to aspects of the present invention;
Figs. 19a-c are views of another example expandable tip design according to aspects of the present invention;
Fig. 20 illustrates an example expandable tip and catheter support tube enclosed in a polymeric cover according to aspects of the present invention;
Figs. 21a-c show different possibilities for the distal profile of a polymeric cover for an expandable tip according to aspects of the present invention;
Fig. 22 is a view of another example of a polymeric cover with a planar face and distal rib according to aspects of the present invention;
Figs. 23a-d illustrate isometric and profile views an expandable tip with distally unconnected petals according to aspects of the present invention;
Figs. 24a-d show views of another expandable tip according to aspects of the present invention;
Figs. 25a-d are views of another example expandable tip according to aspects of the present invention;
Figs. 26a-d are views of alternative expandable tip design according to aspects of the present invention;
Figs. 27a-d show views of another expandable tip according to aspects of the present invention;
Figs. 28a-d are views of an expandable tip with independent longitudinal arms according to aspects of the present invention;
Figs. 29a-c are views of alternative expandable tip according to aspects of the present invention;
Figs. 30a-d show views of another expandable tip according to aspects of the present invention;
Figs. 31a-b illustrate views an expandable tip with a mesh construction according to aspects of the present invention;

### Detailed Description

The objective of the disclosed designs is to create an expandable mouth for a clot retrieval catheter capable of providing both local flow restriction/arrest with a large distal facing mouth that is tailored to provide sufficient radial force and high flexibility to be capable of navigating tortuous areas of the vasculature within an outer catheter to reach an occlusive clot. The large mouth designs offer substantially greater aspiration efficiency and flow restriction capabilities. Such advantages can also be especially beneficial in the case of stroke intervention procedures, where vessels in the neurovascular bed are particularly small, circuitous, and fragile. As a result, a tailored axial and bending stiffness profiles of the expandable mouth tip can inhibit kinking and binding while tracking through these vessels. The tip can have a collapsed state so the clot retrieval catheter can be compatible with relatively low-profile access sheaths and outer catheters, so that a puncture wound in the patient's groin (in the case of femoral access) can be easily and reliably closed. The expandable mouth can also feature internal and/or external low-friction liners, and an outer polymer jacket or membrane disposed around the supporting structure. These improvements can lead to safe and more rapid access of a catheter and other devices to complex areas in order to more reliably remove occlusions and shorten procedure times.

Another advantage of using and having a clot retrieval catheter with an expanding mouth delivered through an outer catheter is that once the clot has entered the distal end of the clot retrieval catheter, the clot retrieval catheter can be retracted through the outer catheter such that the outer catheter is left in place to maintain access at the target treatment location. While it is appreciated that certain clots may also require that the outer catheter be retracted with the clot and inner clot retrieval catheter, the majority of clots are likely to be removed through the inner clot retrieval catheter. With this combination there will be greater confidence that the lumen of the outer catheter is clean of debris for reduced risk during contrast injection that potential thrombus remnants may be dislodged from the catheter. With traditional catheters, a user would often have to remove the outer catheter to flush any thrombus remnants outside of the body prior to injecting contrast, at the cost of losing access to the target treatment location. The present invention provides means to minimize the number of catheter advancements required to treat a patient, thereby reducing the likelihood of vessel damage and the associated risk of vessel dissection in cases where multiple passes are required.

Specific examples of the present invention are now described in detail with reference to the Figures. It should be appreciated that when used herein, tip framework, mouth framework, support frame, etc. are interchangeable and all refer to the same structure. The designs can often have a polymeric membrane cover, which is typically not shown for clarity of the underlying framework. While the description is in many cases in the context of mechanical thrombectomy treatments, the designs may be adapted for other procedures and in other body passageways as well.

Accessing the various vessels within the vascular to reach a clot, whether they are coronary, pulmonary, or cerebral, involves well-known procedural steps and the use of a number of conventional, commercially available accessory products. These products, such as angiographic materials, rotating hemostasis valves, delivery access catheters, and guidewires are widely used in laboratory and medical procedures. When these or similar products are employed in conjunction with the disclosure of this invention in the description below, their function and exact constitution are not described in detail.

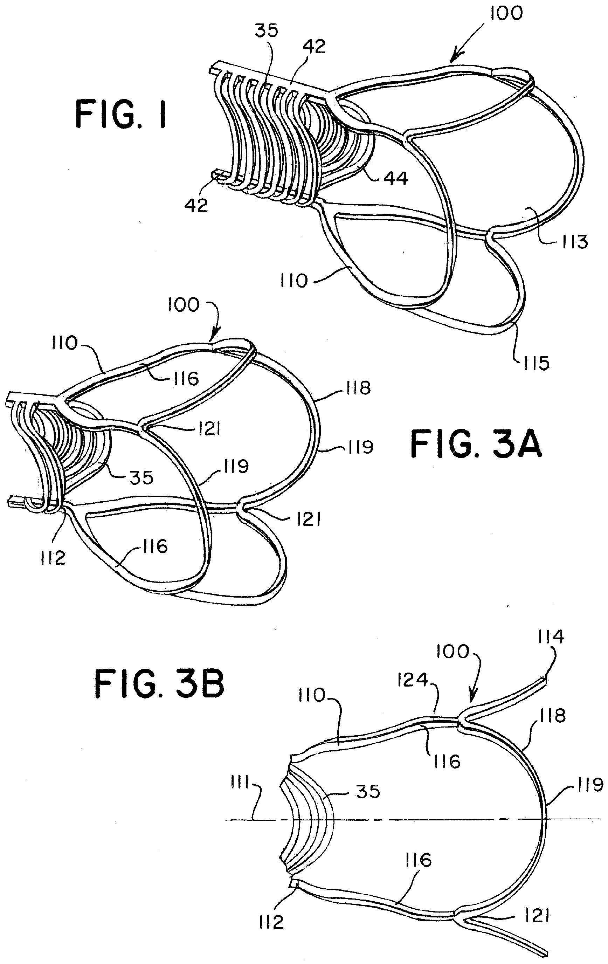

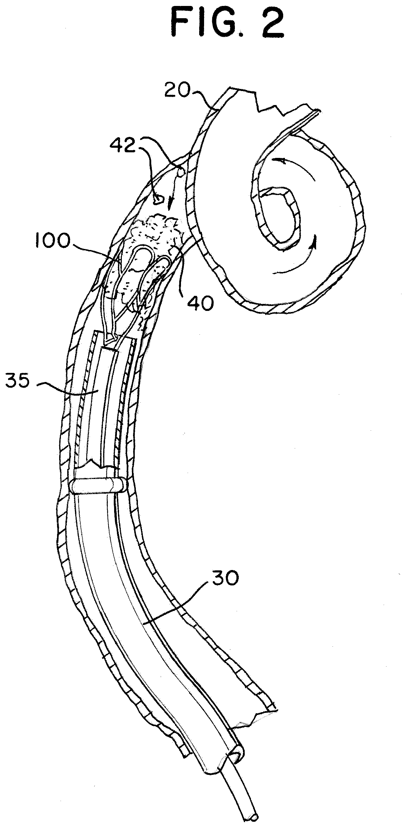

Referring to Fig. 1, a clot retrieval catheter can have a proximal tubular portion 35 and a distal expandable tip 100 which radially expands upon exiting the outer or intermediate catheter in which it is delivered. The tip 100 provides a large distal mouth 113 for aspirating, including capturing a clot 40 or thrombus, sized to have an expanded diameter nearly the same or just larger in diameter than the expected upper end of the target vessel diameter when unconstrained as further illustrated in Fig. 2. When deployed, the mouth 113 of the tip 100 can thus match the vessel 20 diameter and have the radial force to seal with the vessel, or create enough of a flow restriction such that when aspiration (as indicated by the arrows) is applied blood and the clot distal of the mouth will be drawn in to the catheter rather than blood proximal of the tip. If the expanded tip does not seal, or forms only a partial seal, then the suction applied to the clot can be less effective as the flow will be directed proximal of the tip to areas of the vessel and outer catheter 30 which will likely be less restricted. However, even a partially sealing expandable tip 100 will still out-perform many current aspiration catheters that leave more cross-sectional area open to the vessel proximal of the tip. In other examples, an enlarged catheter body support tube 35 or a dedicated seal (not shown) can also be used to occupy the lumen between the clot retrieval catheter and the outer catheter 30.

The support tube 35 of the clot retrieval catheter can be of many different configurations. The support tube 35 can have one or more axial spines 42 extending the length of the support tube. For example, the support tube 35 illustrated in Fig. 1 has two spines spaced 180 degrees apart. The spine or spines can be of tubular or wire construction so as to yield good axial stiffness for advancing and retracting the catheter while having excellent lateral flexibility for navigating within the vascular. The use of multiple spines can encourage flexing along defined planes and while reducing the possibility of the support tube 35 elongating under tensile loads, such as when the expandable tip 100 is withdrawn into the mouth of an outer catheter. Running the length of the axial spine or spines can be a supporting structure with series of ribs or a tubular mesh or braid defining the inner lumen 44 of the support tube 35. The support structure can be a simple circular configuration as shown or take a more complex shape as required, the example shown having an axially curved profile where connected to the spines 42. A substantially cylindrical support tube 35 that does not have a planar cross section can have the ability to expand under compression during retraction of a clot, even if covered by an elastomeric cover or jacket, allowing the tube to "swallow" dense clots that may otherwise be restricted from entering a non-expandable lumen.

The support tube 35 can be formed from laser-cutting a hypotube or other tube stock, or of otherwise similar construction including a braid with overlaid or interwoven spine(s). This enables the support tube 35 to have good push and torque characteristics, small bend radii, kink resistance, and solid resistance to tensile elongation. Commonly used materials include Nitinol and familiar medical-grade stainless-steel alloys like 304 and 316. Hypotubes of different materials, such as stainless-steel for the proximal section of the tubular support and Nitinol for a distal portion of the tubular support tube and for the expansile mouth, said different materials being joined by welding, bonding or by holding interlocking features in place with inner and/or outer polymer jacket materials.

The funnel design of the expandable tip 100 of the disclosed examples can be an integral lattice laser cut directly and integrally with the support tube 35 of the catheter shaft. Alternately, the expansile tip lattice can be injection molded support or mesh frame constructed as a single piece and attached to the support tube through heat welding, adhesives, or similar means. The tip can be designed to expand to a wide range of target vessel diameters, such as a carotid terminus (3.2-5.2 mm), a horizontal M1 segment of the Middle Cerebral Arteries (1.6-3.5 mm), and/or the Internal Carotid Artery (ICA, 2.7-7.5 mm). If the catheter is then retracted from an M1 segment to the ICA (or another route with a proximally increasing vessel inner diameter), the expandable tip 100 will continue to seal the vessel across a range of vessel sizes. Further, a tip capable of a range of target vessel diameters can also seal at vessel bifurcations which can have a wider cross-sectional area than the vessel proximal and vessels distal to the bifurcation. Preferably, the expandable tip 100 of the catheter is expanded at the treatment location to avoid having to advance the expanded tip through the vasculature.

Arranging the expandable tip 100 to have connections in-line with one or more of the spines 42 of the support tube 35 allows advancement forces to be directly transmitted through the spines to the tip during advancement through an outer catheter for enhanced pushability. An in-line connection can also allow other circumferential portions of the support tube 35 to be kept free of joints to adjacent ribs or the tip in order to limit effects on the deliverability of the catheter through increased friction with the outer catheter.

The catheter can also have a cover or membrane (not shown) disposed around or encapsulating at least a part of the support tube 35 and expandable tip 100. Suitable membrane materials can include elastic polyurethanes such as Chronoprene, which can have a shore hardness of 40A or lower, or silicone elastomers. A single- or variable-stiffness cover can be extruded or post-formed over the support tube 35 and tip 100. The cover can also be laminated, or heat welded to the structure.

Alternatively, the cover can also be a formed from a series of polymer jackets. Different jackets or sets of jackets can be disposed at discrete lengths along the axis 111 of the support tube 100 in order to give distinct pushability and flexibility characteristics to different sections of the tubular portion of the catheter. By configuring the jackets in an axial series, it is possible to transition the overall stiffness of the catheter from being stiffer at the proximal end to extremely flexible at the distal end. Alternately, the polymer jackets of the cover can be in a radial series disposed about the support tube in order to tailor the material properties through the thickness. In a further example, transitions between jackets can be tapered or slotted to give a more seamless transition between flexibility profile of abutting jackets in longitudinal series.

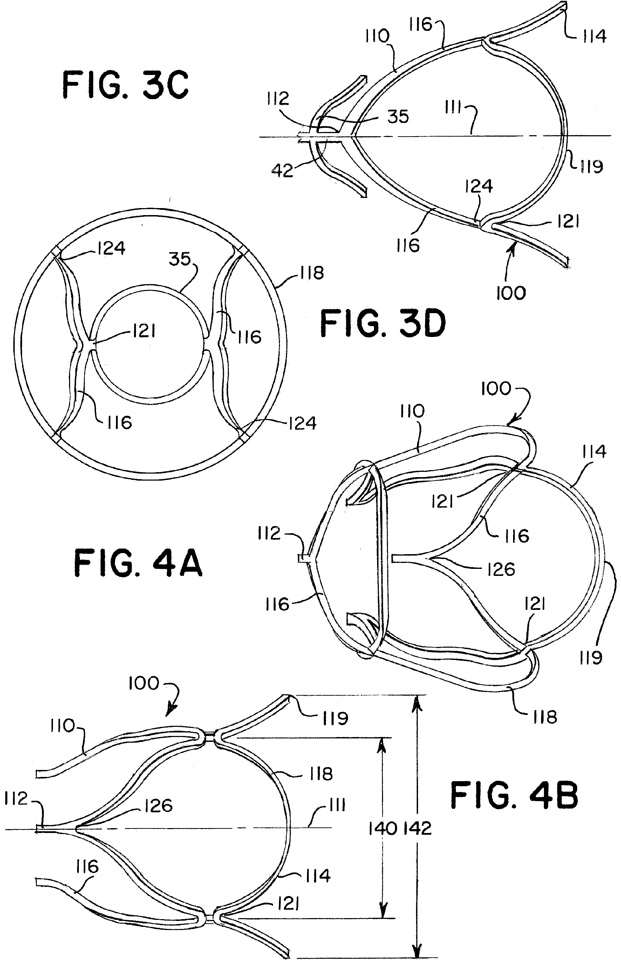

In an example shown in Figs. 3a-d, the expandable tip 100 from Fig. 1 can have a mouth support frame 110 of struts with four distal hoops 118 and two support arms 116. The distal hoops 118 can have four proximal hoop troughs 121 and four distal peaks 119. The support arms 116 can contour the funnel shape of the tip 100, and when expanded and unconstrained, the diameter of the tip can range from 1 mm to 10 mm, or more specifically from 2.5 mm to 7.0 mm for a device intended to treat blockages in the ICA, Carotid Terminus, M1 and M2 locations. The support arms 116 can also facilitate having distal hoops 118 with four proximal hoop troughs 121 that are fully connected to the support arms, thereby eliminated the risk of the hoop troughs becoming snagged on the mouth of an outer catheter during retraction of the expanded mouth.

The support arms 116 can be V- or Y-shaped struts extending from the support tube 35 merging to two connections to the spines 42 or the support tube at the proximal end 112 of the expandable tip 100 and four connections to the distal hoops 118. The V- or Y-shaped support arms 116 can give the frame 110 similar support to a device with four or more support arms, while reducing the number of connections to the support tube 35. Having two connections to the support tube spaced 180 degrees apart allows the support frame 110 to hinge about the connections when collapsed within an outer catheter during advancement through tortuous vasculature, or when deployed in curved vessels to fully appose the vessel walls with the hoops 118. The hinging action biases bending along a plane extending radially through the two connections and the longitudinal axis 111 of the frame 110. It can be appreciated that support frames 110 with two connections to a support tube 35 can have additional support arms extending from a single connection where distal hoops 118 with more than four proximal troughs 121 are used.

The stiffness and changes in stiffness for the mouth support frame 110 is important in situations where distances and tortuosity can be significant, such as when it must be advanced from a patient's inner thigh, over the cardiac arch, and up into the neurovascular inside the skull. To further tailor the stiffness, the strut width of the support arms 116 and distal hoops 118 can be varied along the length of the strut by incorporating one or more narrowed segments 124. For example, greater width at the peak of a V-shaped support arm 116 can give greater radial force capability while narrowed mid-sections can help to reduce lateral stiffness to aid in bending to track through tortuous vasculature. Having a greater width adjacent to the proximal troughs 121 of the distal hoop 118 can also give increased radial force. Narrowed segments 124 at the distal hoop peaks 119 can soften the distal end of the expandable tip 100 to improve the atraumatic characteristics of the tip, and the narrowed segments 124 can also aid the hoop 118 in collapsing back into the mouth of an outer catheter by reducing the force required to collapse the distal peaks 119 of the frame 110.

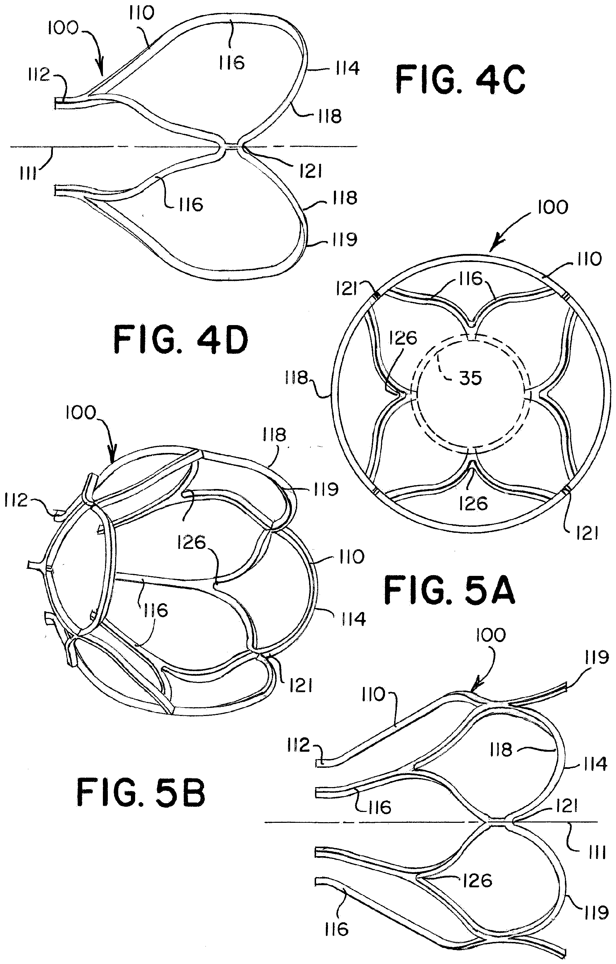

Turning to Figs. 4a-d, a support frame 110 can have an array of four Y-shaped support arms 116, each with two support struts, and four distal hoops 118 with four hoop troughs 121 and four distal hoop peaks 119. An arrangement where each support arm 116 has two supporting struts allows for greater support for the cover or outer jackets while limiting the number of distal peaks 119 to four, allowing an atraumatic larger, more rounded contour for the distal hoop 118. Having four connections between the support tube 35 and the proximal end of the 112 of the mouth framework with four support arms 116, as shown in Fig. 4a, can give the joint greater axial stiffness over a tip with only two connections in instances where the tip 100 needs to be advanced in the expanded configuration. The curvature of the support arms 116 can also allow the frame 110 to lengthen and shorten at opposite sides when collapsed in an outer catheter for enhanced trackability through tortuous blood vessels.

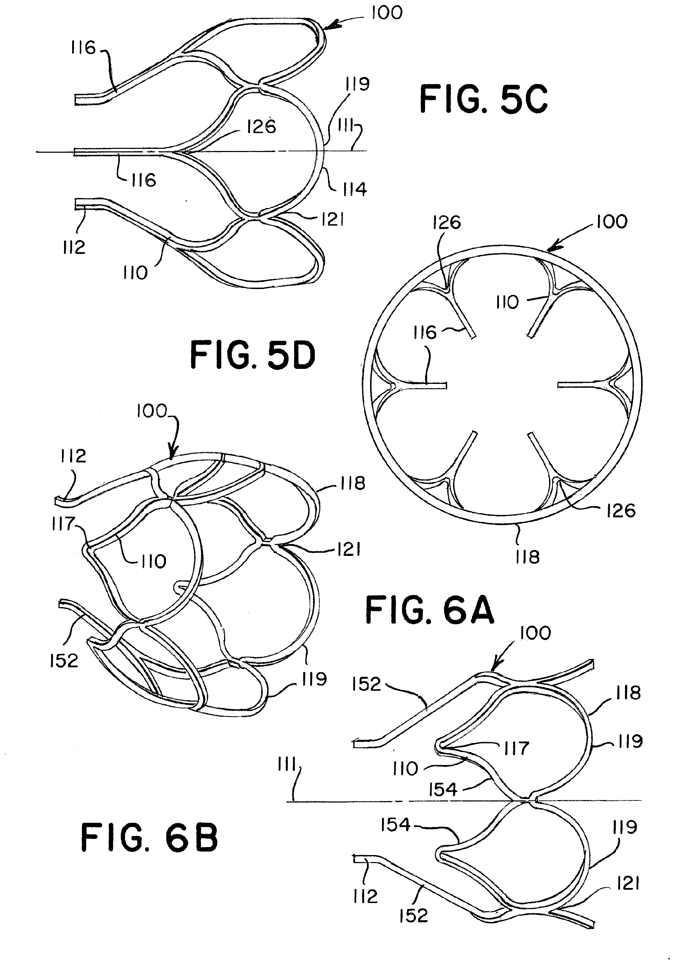

Another support frame 110 can have six Y-shaped support arms 116 and six hoops 118 with six distal peaks 119, as shown in Figs. 5a-d. It can be appreciated that the number of distal peaks 119 and support arms 116 employed can vary from 1 to 50 or more. A configuration with an increase in the number of distal peaks 119, hoops 118, and/or support arms 116 can yield increased support for an outer cover or jacket an increased lateral frame stiffness and radial force. Similar to other examples, sections of the frame 110 can have struts of variable width to tailor the desired flexibility characteristics and bias bending at certain points on the frame.

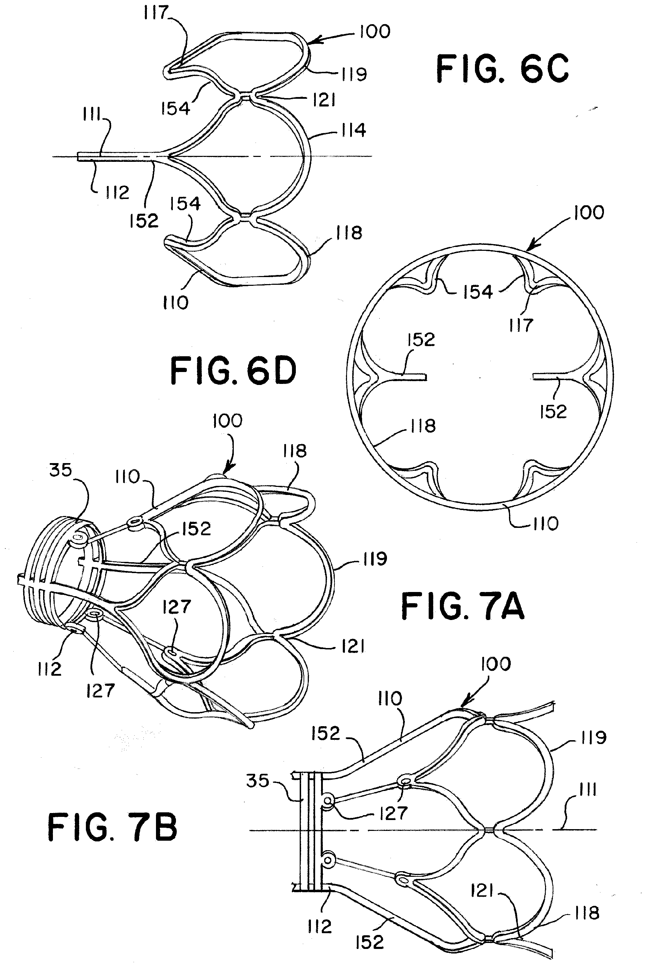

Figs. 6a-d shows another example of a support frame 110 for the expandable tip 100 which can have six hoops 118, six distal peaks 119, and six support arms. Two of the support arms can be Y-shaped support arms 152 with connections to the support tube 35 at the proximal end 112 of the frame 110, while the other four arms can be V-shaped support arms 154 which can terminate at a peak 117 proximal to their respective hoop support troughs 121. This design would provide a frame 110 with closed cell support hoops 118 to enhance the radial force capabilities of the tip 100 while having only two connections to the support tube 35. Having the two connections with the support tube 35 spaced 180 degrees apart allows the frame to hinge about the supports on a plane that extends radially through the Y-shaped supports 152 and the longitudinal axis 111. This hinging allows for enhanced trackability through an outer catheter and improved conformability to the walls when a deployment location is a curved vessel.

The V-shaped support arms 154 provide additional surface area to support an outer cover or membrane (not shown). The outer membrane can reduce the likelihood of the proximal peaks 117 of the V-shaped arms 154 of snagging on branching vessels or the mouth of an outer catheter during retraction. In another example, the proximal peaks 117 of the arms 154 can be more rounded or U-shaped similar to the distal peaks 119 of the support hoops 118.

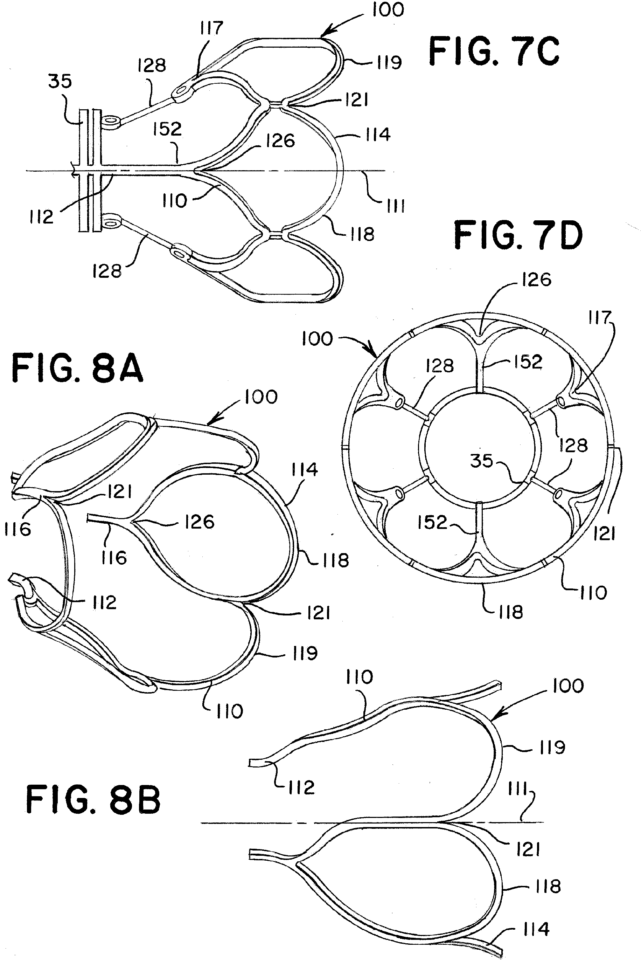

In another example, a mouth support framework 110 for a clot retrieval catheter can have six distal hoop peaks 119, two Y-shaped support arms 152, and four V-shaped support arms 154, as seen in Figs. 7a-d. The V-shaped support arms 154 can have eyelets 127 at their proximal peaks 117 aligned longitudinally with eyelets of a support tube 35. An array of link members 128 can extend between the eyelets 127 and connect to respective eyelets using knots, welding, or plastic deformation to form a bulb, loop, or enlarged abutment. As an alternative, polymeric members can utilize heat-forming processes to form the bulbs. The link members 128 can be string-like members, chain-link members, rigid or semi-rigid members, or a combination thereof. Alternately, or in addition to, radiopaque markers (not illustrated) can be disposed in the eyelets 127 to help with the positioning of the mouth 110.

Similar to other examples, this design can provide a closed-cell structure for the distal hoop 118 to provide enhanced radial force while the eyelets 127 and link members 128 mean there are only two rigid connections with the Y-shaped arms 152 to the support tube at the proximal end 112. Spacing the connections between the support tube 35 and Y-shaped arms 152 at diametrically opposed positions of the tube allows the frame 110 to hinge about the connections for trackability through an outer catheter and to better conform when the target location is in a curved vessel.

The array of link members 128 reduce the likelihood of the free proximal peaks 117 of the V-shaped support arms 154 snagging on the mouth of an outer catheter when the expandable tip 100 is retracted. The connections of the link members to the eyelets 127 at the proximal peaks 117 of the V-shaped arms 154 and the eyelets of the support tube are not rigid, allowing the members to move through the eyelets 127 of these connections in a loose manner so the support frame 110 can flex easily in tortuous vessels. If the link members 128 are made flexible, they will not contribute to the bending stiffness of the tip 100 in a collapsed state but can become taut once the distal support hoop 118 is expanded. Flexible members can be sufficiently tough to support an outer cover or membrane to counteract the negative pressure forces exerted on the cover during aspiration.

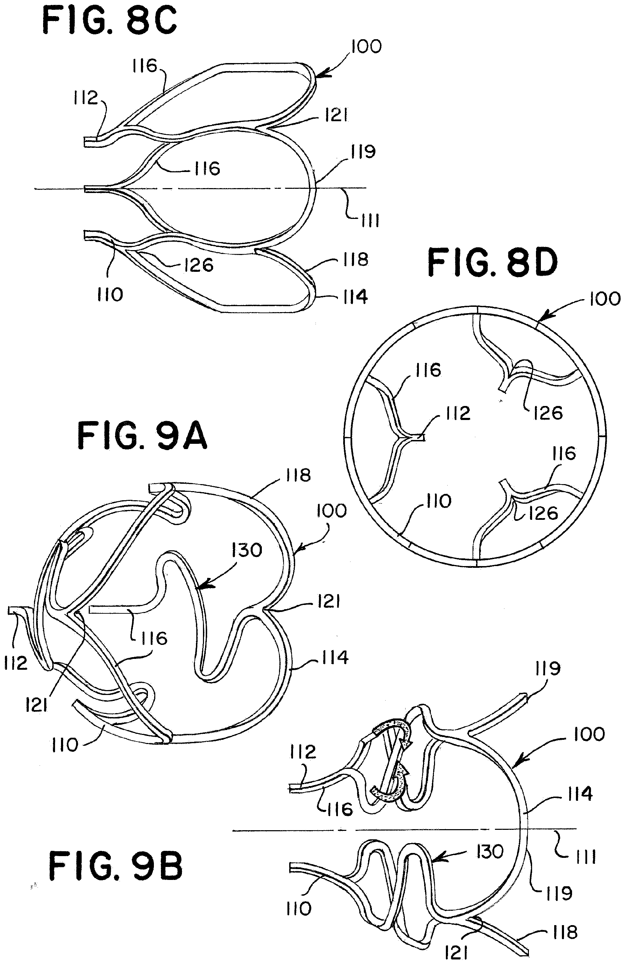

Another mouth frame 110 example can have distal support hoops 118 with six peaks 119 around the perimeter of the mouth, as illustrated in Figs. 8a-d. The frame 110 can have three Y-shaped support arms 152 connecting adjacent hoop troughs 121 with the support tube 35. Though similar in some ways to previously described examples, an expandable tip 100 with three support arms will be more flexible in the collapsed delivery configuration than a tip with more arms. Having fewer connections to the support tube can also aid the frame 110 in collapsing back to a constrained state on retraction.

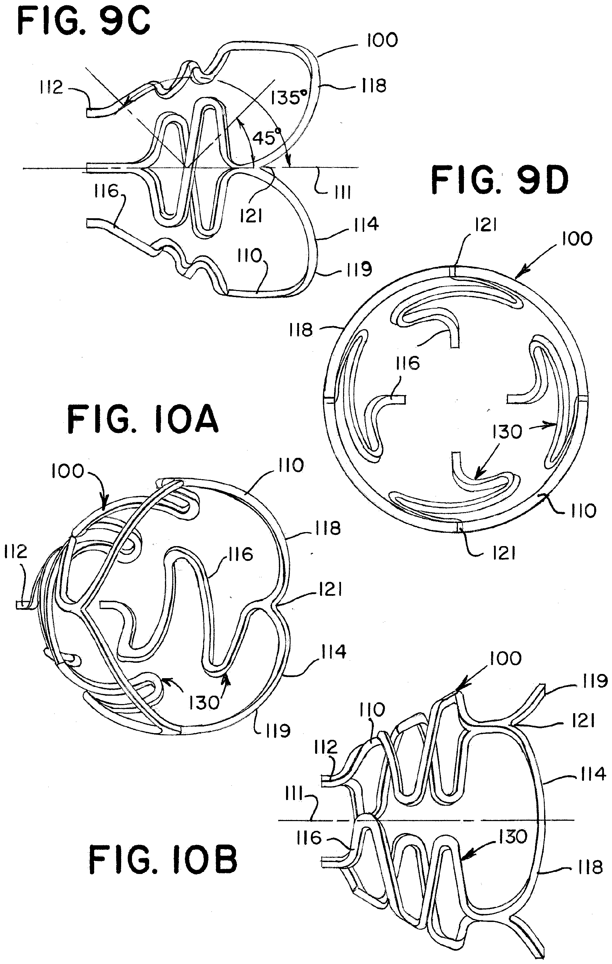

Figs. 9a-d shows another support frame 110 with four distal hoops 118 with four rounded distal peaks 119 and four hoop troughs 121. The struts of the distal hoops 118 can be tangentially aligned with four support arms 116 extending to a support tube 35 from the respective hoop troughs 121. The support arms can have a circumferential undulations or a sinusoidal waveform pattern 130 along at least a part of their length, where taken together the support arms define a substantially conical profile for the support frame 110. The sinusoid pattern 130 can be arranged so that when the tip 100 is in a collapsed configuration, the local peaks of adjacent support arms 116 do not overlap and instead can nest together to fold the frame in a complimentary way. The amplitude of the waveform pattern 130 can be selected so as to be large enough to provide generous support the polymeric cover or outer jacket while not being too large as to require the support arms 116 to elongate longitudinally in order to collapse neatly within an outer catheter. The waveform patterns 130 give the frame 110 the flexibility to shorten and lengthen in various directions as the tip is being tracked through an outer catheter in tortuous vessels, or to conform with and achieve full apposition with the vessel walls when deployed at a target location with complex geometry.

For optimized lateral flexibility, the support frame 110 can be constructed so that the struts forming the waveform pattern 130 of the support arms 116 form an angle between 45 degrees and 135 degrees with respect to the central longitudinal axis 111 when the frame is in a flat pattern or plan view. In this configuration, the more longitudinally oriented portions of the support arms 116 can bias bending about the struts extending between the peaks of the waveform pattern 130 when the frame is subjected to torsional moments, making the support arms more reliant on torsional flexibility than lateral flexibility. It can be appreciated that the angle formed by the struts of the pattern can fall outside the 45-135-degree range if it is desired to trade some torsional flexibility for additional lateral flexibility in bending.

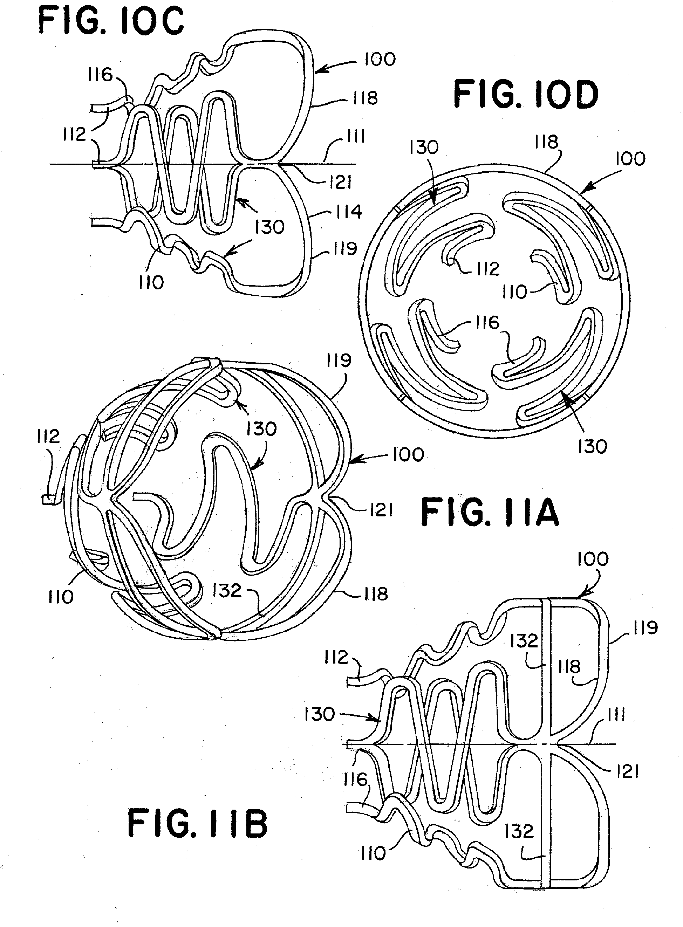

In a similar example seen in Figs. 10a-d, a support frame 110 can have distal hoops 118 with four rounded distal peaks 119 and four support arms 116 with sinusoidal waveform patterns 130 extending from hoop troughs 121. Compared to the example in Fig. 9a, the support arms 116 can have additional local peaks in the wave pattern 130 to enable the frame to have less of an effective gap between adjacent support arms and provide additional support for a flexible outer membrane or cover. Additional undulations in the pattern also yields more strut length over which torsional and lateral strains can be distributed when the frame 110 is in bending while being tracked through an outer catheter.

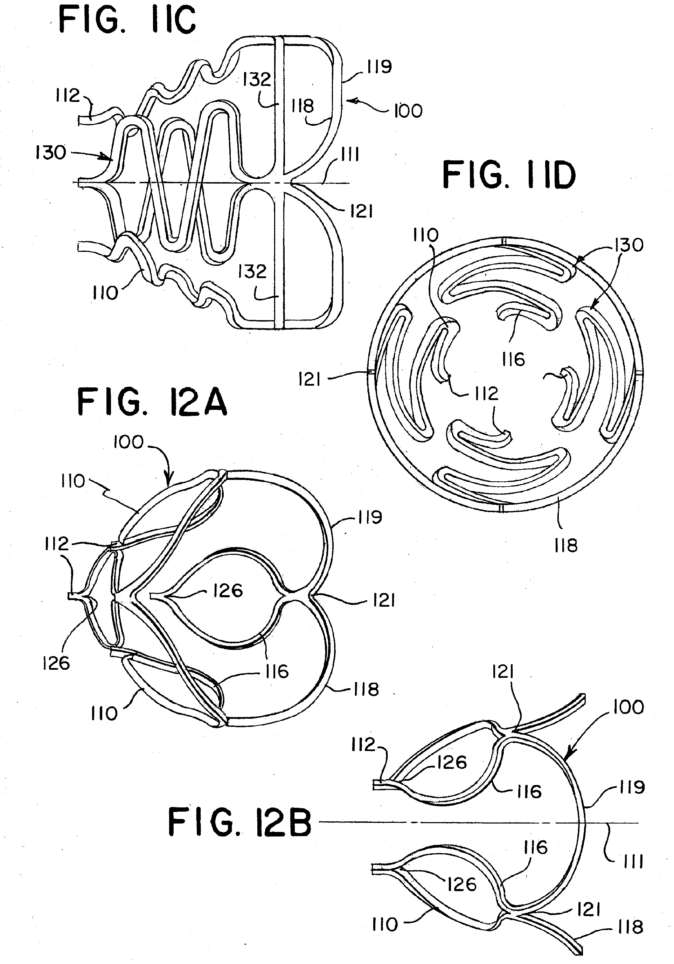

Another support frame 110 of an expandable tip 100 similar to that of Figs. 10a-d is shown in Figs. 11a-d. The frame can have four distal hoops 118 and four support arms 116 with sinusoidal wave patterns 130 along at least a part of their length. As described, the number of peaks and the amplitude of the undulations of the wave pattern 130 can be tuned for the desired flexibility characteristics of the frame 110. In addition, the width of the struts can be adjusted to maintain flexibility while achieving a desired radial force.

The frame 110 can have an additional strut or struts acting one or more torsional members 132 extending between proximal hoop troughs 121 of the support hoops 118. The torsional members 132 can extend in a circular fashion about a central longitudinal axis 111 of the device as shown such that it can move distally under torsional loading during retraction into an outer catheter, This movement can aid the frame 110 in pinching a stiff clot that may be otherwise restricted from entering the catheter lumen 44 fully so that the grip on the clot is secure as the catheter is withdrawn through the vasculature. In another example, a portion of the hoop peaks 119 and/or the torsional members 132 can have an axially rounded or curved profile, or the struts can intersect at an intermediate point to serve as hinges and reduce the transmission of the torsional moment as the frame 110 is collapsed. This reduced moment can be beneficial in devices where the outer cover or jackets are constructed from stiffer materials with reduced elastic strain capacity and elongation at break, as the cover would not be required to move as much as the frame folded or collapsed into an outer catheter.

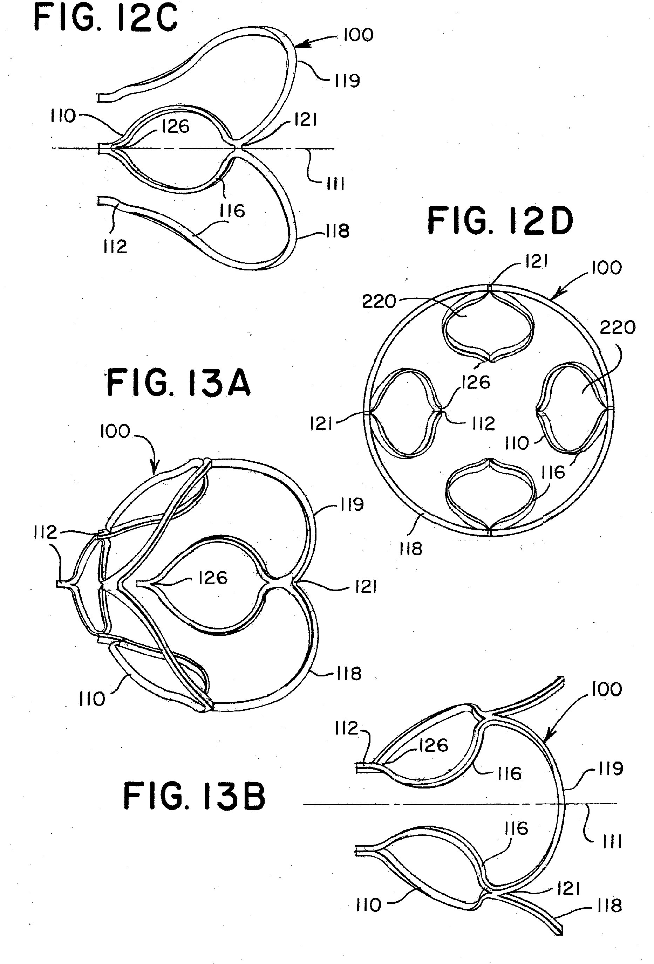

A further example of a support frame 110 having distal hoops 118, four support arms 116, and four hoop peaks 119 is disclosed in Figs. 12a-d. Each support arm 116 can branch between a hoop trough 121 and a more proximal support trough 126 to form an enlarged cell or opening 220. The enlarged openings in the arms 116 can allow the arms to shorten and lengthen on opposing sides around the longitudinal axis 111 of the frame so that the device can track easily through an outer catheter in tortuous vessel paths. The branching of the support arm 116 struts can allow the arms to torque and bend more freely than if a single strut directly linked the hoop troughs 121 with the support troughs 126 without a cell. The j oint at the hoop troughs 121 can also be longer axially than shown in Fig. 12c to better distribute lateral and torsional strains when the frame 110 is required to flex between hoop peaks 119. Similarly, the enlarged opening can also have a lesser or greater spacing than that shown to further encourage the independent motion of the support arms 116 with respect to each other.

The struts of the distal hoops 118 can also have a different width than those of the support arms 116. For example, wider or thicker struts of the hoop approximate the hoop peaks 119 can provide the support frame 110 with greater radial force capability at the hoop and more flexible support arms. Narrower struts near the peaks 119 can allow the hoops to be more compliant when sealing with the walls of a vessel.

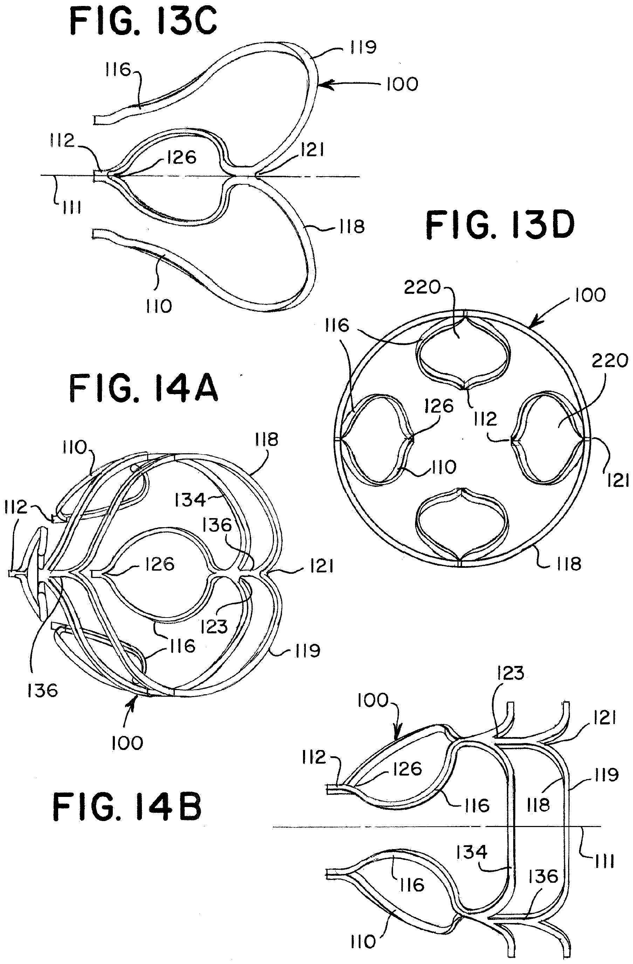

Another support frame 110 having four branching support arms 116 and four hoop peaks 119 is illustrated in Figs. 13a-d. Compared to that in Figs. 12a-d, this design can feature a modified shape for the hoop trough 121 connecting joints, where a direct connection can transfer loads more smoothly between sections of the distal hoop 118 and the enlarged cells 220 of the support arms 116. The shape of the hoop troughs 121 can also be changed to adjust the intersecting angles of the branching struts forming the distal hoop 118 and support arms 116 to tailor the axial stiffness of the support frame 110.

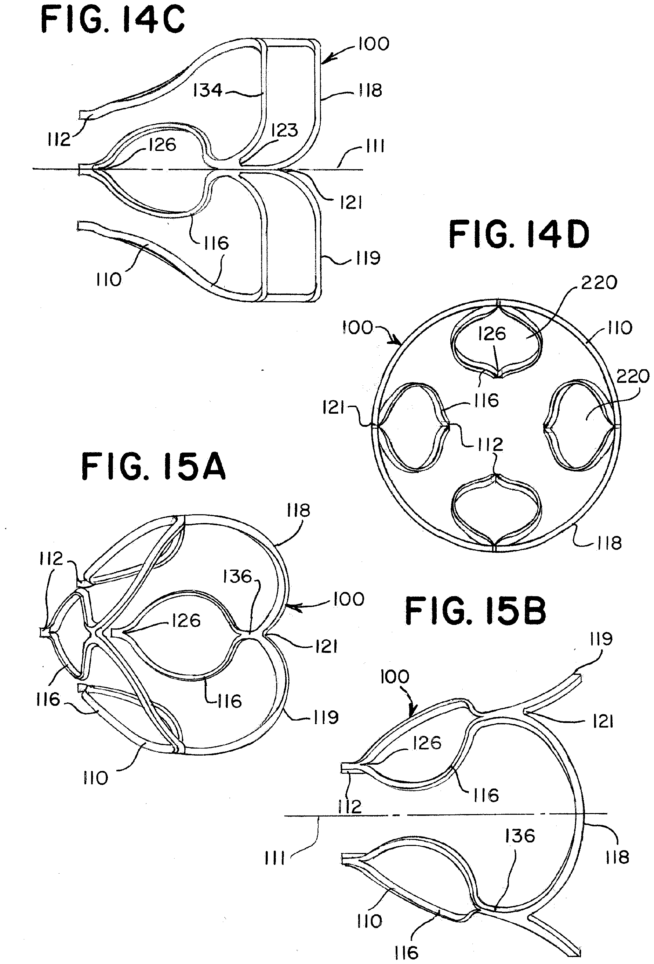

Figs. 14a-d shows another version of a mouth support framework 110 with four support arms 116 having enlarged cell openings 220 and both proximal support hoops 134 and distal support hoops 118. The distal support hoops 118 can have four hoop peaks 119 and join proximally at four respective hoop troughs 121. An array of connector struts 136 can link the more distal hoop troughs 121 with proximal hoop troughs 123 serving as the joint between the struts of the proximal support hoops 134 and the support arms 116. A device having multiple support hoops in an axial series can be capable of exerting a greater radial force for improved apposition and sealing with a target vessel. The seal provided by the expandable tip 100 can allow for more effective aspiration by directing full aspiration power to the portion of the vessel distal of the tip, while maintaining a funnel-shaped profile provides a converging entry for a clot to be progressively elongated to prevent clot shearing and fragmentation.

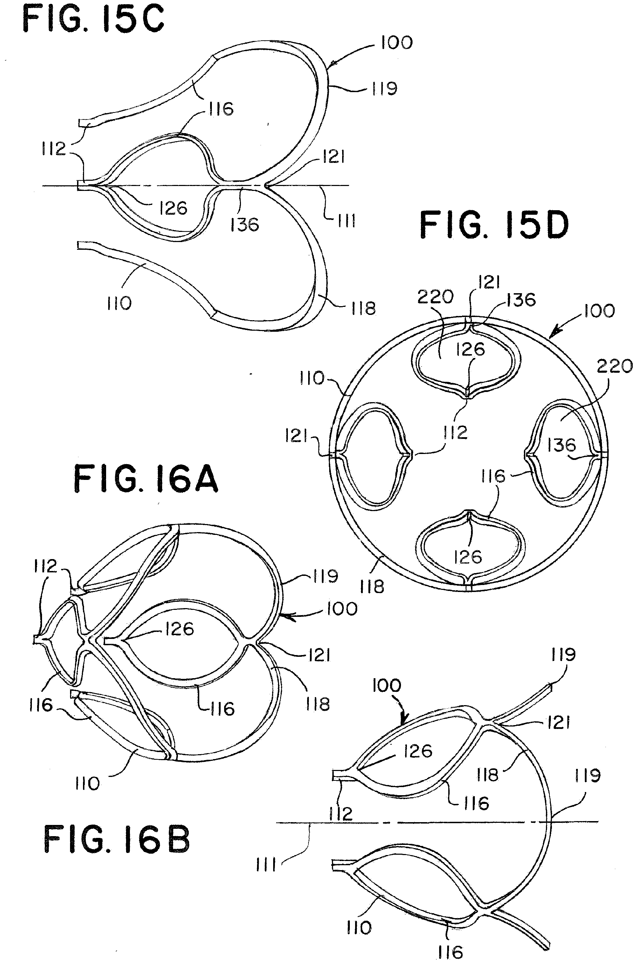

Various views for a design where an expandable support frame 110 has elongated connector struts 136 between the distal hoops 118 and support arms 116 is shown in Figs. 15a-d. The connector struts 136 can extend between the terminations of the enlarged cell opening 220 support arms 116 and the struts of a distal hoop 118. The enlarged openings 220 in the arms 116 can allow the tip 100 to shorten and lengthen on opposing sides when maneuvered in an outer catheter, while having connectors 136 with a greater length can give greater lateral flexibility in bending, allowing the distal support hoops 118 to bend and flex with respect to the support arms in tortuous paths. Further, the length of the connector struts 136 can be varied, or even include curves or undulations, to allow different profiling of the cover or outer jackets so as to provide a more atraumatic interface with the walls of a vessel.

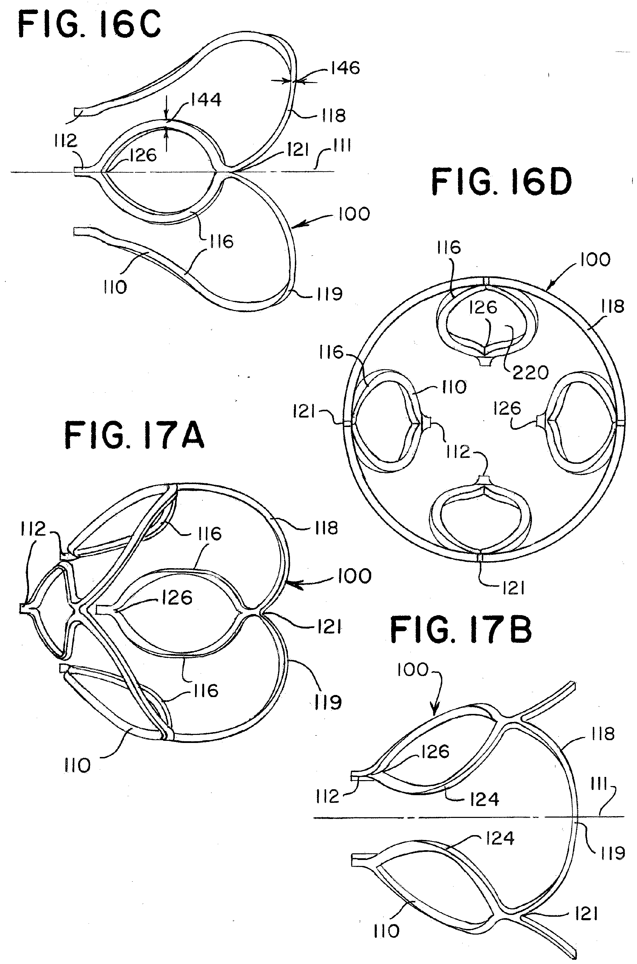

Figs. 16a-d illustrate another support frame 110 variant with a distal support hoop 118 and four support arms 116 with enlarged openings for flexibility. The struts of the support arms 116 can have a first strut width 114 along at least a portion of the length between the support troughs 126 and the hoop troughs 121 different than a second strut width 146 used in the support hoop 118. For example, the second strut width 146 of the hoop can be thinner, reducing the radial force generated by the hoop and providing for more gentle contact with the vessel walls. A thinner hoop can also reduce the friction generated between the hoop 118 and the inner diameter of an outer catheter to allow for easier advancement and retraction of the tip through the outer catheter. Similarly, an increased thickness for the first strut width 114 in the support arms 116 can counteract the reduced radial force of the support hoop 118 by providing added stiffness when the tip 100 is expanded to resist the negative pressures generated during aspiration to keep the frame 110 expanded and apposing the vessel wall. The arms 116 can also have closed cells or undulations to provide more surface to support an outer cover or membrane.

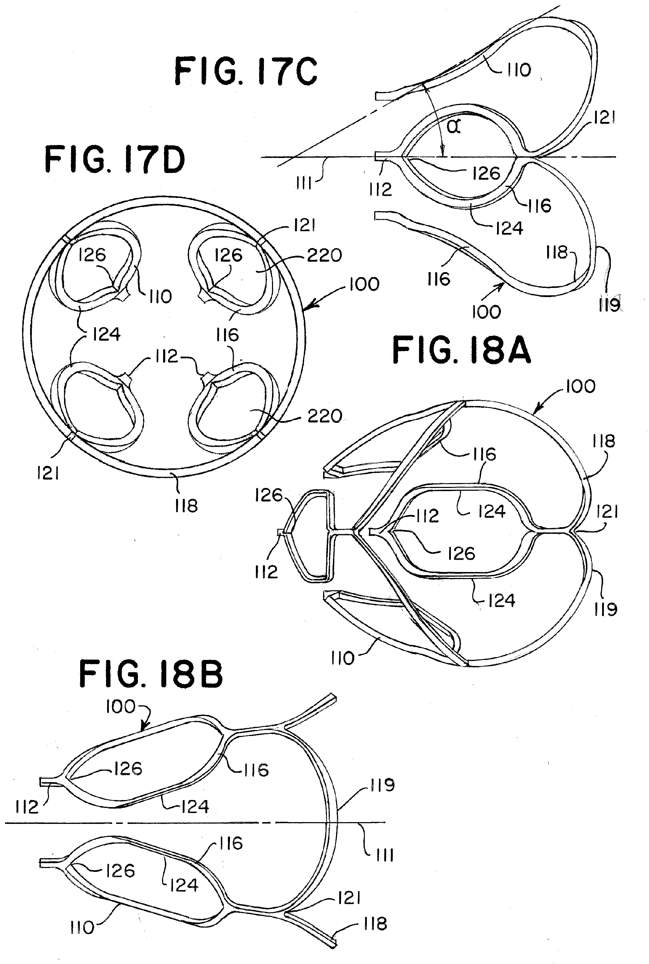

Depending on the location of an occlusive clot and the requisite pushability and flexibility demands on the support frame, other advantages can be gained by varying the strut width of the support arms of the frame. A support frame 110 with four support arms 116 having enlarged closed cell openings and struts of variable thickness can be seen in Figs. 17a-d. The support arm struts can include one or more narrowed segments 124 along their length between proximal support troughs 126 and distal hoop troughs 121. Narrowed segments 124 can enhance the lateral flexibility of the frame 110 when in the collapsed configuration for advancement through an outer catheter, or when deployed within a curved vessel so that the frame can contort while maintaining full apposition to the vessel walls. If the narrowed segments 124 are located mid-span of the support arm 116 struts and away from the support 126 and hoop troughs 121, the frame can remain highly flexible with support for a membrane.

When flexibility characteristics are added to the support arms 116 of the tip framework 110, the radial force capabilities of the tip 100 can be maintained through a more generous support arm angle, α, to the longitudinal axis 111. The radial force can be tuned so that an adequate seal can be maintained without contributing to vessel trauma. For example, when the tip framework 110 is heat set with an angle α equal or greater than 30 degrees, a larger component of the expansion forces can be exerted in the radial direction.

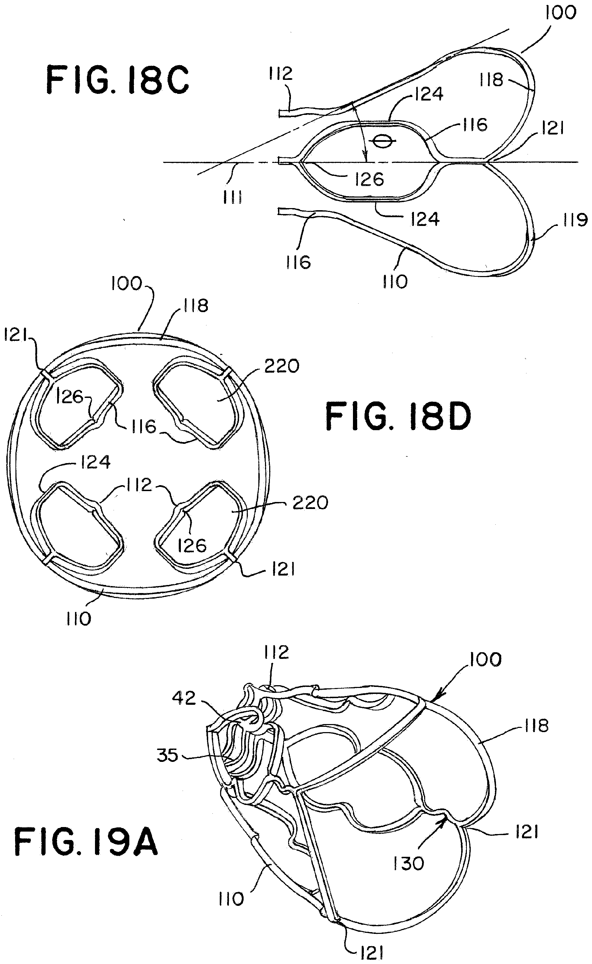

Various views of a similar design for an expanding support frame 110 with a less tapered funnel shape and four support arms 116 with a decreased support arm angle θ is depicted in Figs. 18a-d. Support arm angle θ can be, for example, approximately 30 degrees as opposed to the approximately 45 degrees in Figs. 17a-d. A lower support arm angle can reduce the force required to advance the device through an outer catheter, while also lowering the corresponding force necessary to retract the expanded frame 110 into the outer catheter during a procedure. These forces can be further reduced by incorporating widened or narrowed segments 124 into the arms 116 and/or the distal hoops 118.

Alternatively, the support arm angle θ can be in a range that is less than 45 degrees so that the catheter tip 100 will be less likely to expand further when advanced distally in an outer sheath or blood vessel, but greater than 10 degrees so that the mouth framework 110 of the catheter will be kept relatively short in length. Optimizing the support arm angle θ can also help the tip 100 to seat against the distal tip of an outer catheter and the walls of a target vessel.

When in the expanded state, at least part of the mouth framework 110 may taper distally from a larger peak radial dimension to a smaller radial dimension. In this configuration, the outer axial profile of the tip body can also be broadly rounded to provide a smooth interface with the vessel wall. A portion of the distal support hoops 118 can extend radially inward as shown in Fig. 18d to reduce the likelihood of the distal hoop peaks 119 pressing into the vessel walls and allow the expanded tip to be advanced through a vessel for short distances without causing vessel damage.

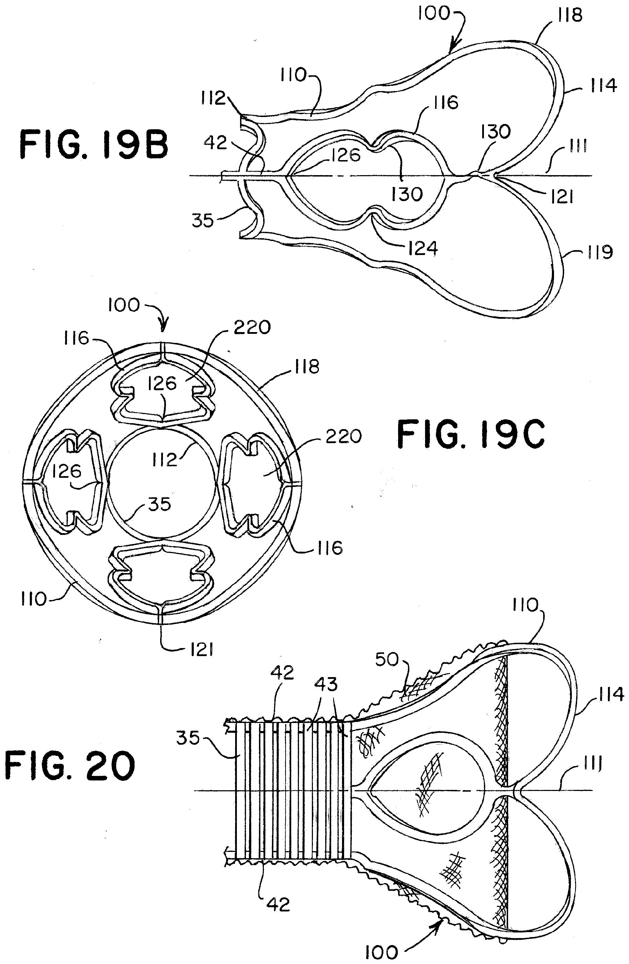

Figs. 19a-c shows another support frame 110 of an expandable tip 100 connected to a support tube 35 with four support arms 116. This frame 110 can also have a support arm angle θ that is less than that of previously seen designs. Portions of the distal support hoops 118 can extend radially inward between adjacent hoop troughs 121. The support arms 116 can be clocked 90 degrees apart around the longitudinal axis 111 and can branch to form one or more closed cells, allowing the frame to lengthen and shorten on opposite sides while navigating through tortuous areas of the vasculature. Additionally, the support arms 116 can include one or more waveform patterns or undulations 130 between the proximal end 112 and distal end 114 of the tip, including portions of the closed cells or segments adjacent to the more proximal support troughs 126 and the more distal hoop troughs 121 as shown. The undulations 130, combined with narrowed segments 124, can enhance the lateral flexibility of the support frame.

The support arms 116 can be connected at the proximal end 112 of the mouth frame 110 with the support tube 35 of the catheter. One or more spines 42 of the support tube can be aligned with one or more of the support arms to allow for the smooth transmission of longitudinal forces between the spines and the frame. Support arms 116 not aligned with spines 42 can link with support arms that are aligned or terminate at points approximate the distal end of the support tube 35. In thus configuration, compressive forces generated during clot retrieval may not cause unwanted expansion of the support tube 35 or mouth frame 110 during a procedure.

Any of the mouth support frames 110 disclosed herein can be enclosed or encapsulated by an elastomeric membrane cover 50. While many of the previous figures have not shown the cover for clarity, Fig. 20 shows how a membrane 50 can cover at least a part or all of the struts of the mouth framework 110 of the expandable tip 100 and at least a part of circumferential ribs 43 and axial spines 42 of a support tube 35.

The thickness of the membrane cover 50 can be maintained between and over the struts of the mouth support frame 110 or it can vary in thickness along the frame 110. In one example, the cover 50 can be applied where the thickness of the membrane between struts is close to the ligament thickness of the membrane above and below the struts. In another, the cover can have a uniform wall thickness where the thickness between the struts is greater than the ligament thickness remaining above and below the struts. The membrane cover 50 can also include geometric features and/or thinned regions positioned appropriately to vary the contribution of stiffness from the membrane to the overall assembly of the support frame 110 and cover together.

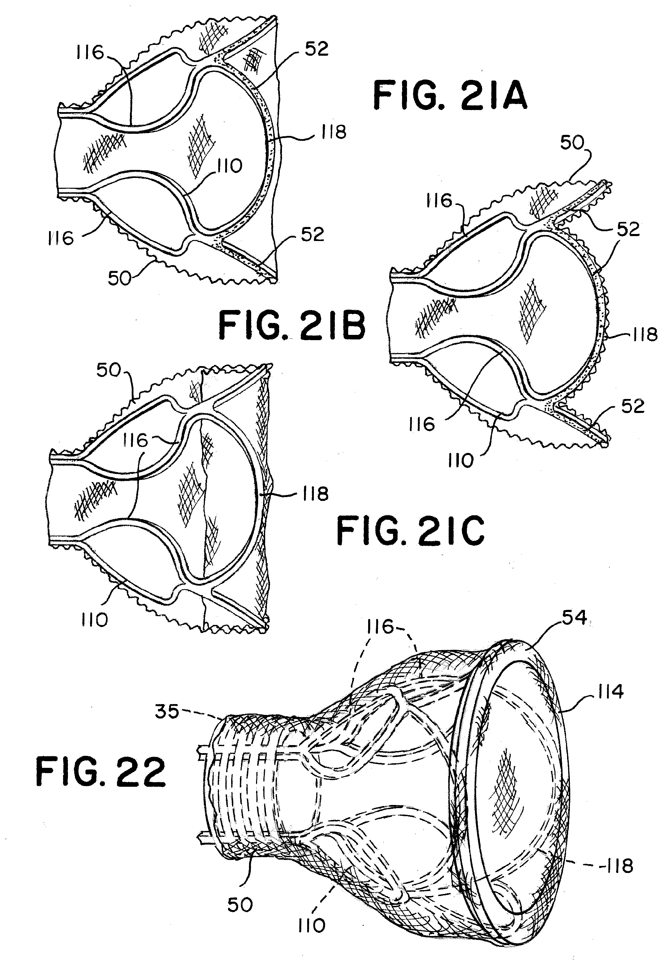

The membrane cover 50 can be stitched to the struts of the support tube 35 and mouth frame 110, or it can be reflowed over and between struts (to be flush with the inner surface of the support struts, or an inner layer and an outer layer may be utilized so that the struts have membrane material above the outer surface and below the inner surface of support struts), heat shrunk and bonded to the outer surface of support struts, or welded in place in defined zones 52. The cover 50 can also be formed through a dipping process (to be flush with the inner surface of the support struts, or an inner layer and an outer layer may be utilized so that the struts have membrane material above the outer surface and below the inner surface of support struts). How the cover 50 conforms to the distal end 114 of the mouth framework 110 can also be varied, as seen in the profile examples of Figs. 21a-c. The membrane cover 50 can be finished with a planar face (Fig. 21a), or the cover can be trimmed to follow the contours of the support frame 110 struts and heat welded in a zone 52 along the perimeter of the distal hoop 118 (Fig. 21b). The heat can locally reflow and/or bond the membrane to the support struts and can at least be bonded in the region of the hoop peaks 119 of the distal hoop 118. In another example in Fig. 21c, the cover 50 can be loose or baggy enough to be folded radially inward (or radially outward) to a position proximal of the distal end 114 of the mouth framework hoop struts 118 and heat welded or otherwise bonded between the inner and outer layers. The membrane may extend fully within the frame so that no frame struts are exposed.

The membrane cover 50 can be of a construction where it has good ductility and a high elastic strain limit so that it can be easily expanded by minimal radial forces from the underlying support frame 110, as shown in Fig. 22. Or, if the cover 50 is formed when the frame in the expanded configuration with an elastomeric or non-compliant material, it can be capable of wrapping down neatly when collapsed for delivery and recovering when expanded for use. The membrane cover 50 can be formed with a circular mouth and may include a soft elastomeric or gel rib 54 (formed through reflowing, dipping, or molding processes) to provide atraumatic contact with a vessel wall as shown. The cover of the mouth support frame 110 can also have flow directing features, such as a plurality of flexible fins, vanes, or recesses disposed around the outer and/or inner circumference in a configuration that entrains vortex or laminar flow. Such features can be included in a forming or molding mandrel.

To allow for smooth delivery of the clot retrieval catheter through an outer catheter, and auxiliary devices through the lumen of the clot retrieval catheter, the expandable tip and/or the outer surface of the membrane or outer jackets can be coated with a low-friction or lubricious hydrophilic material, or a low friction material such as a fluoropolymer like PTFE or FEP. Additionally, the inner surfaces of the struts of the expandable tip, or the inner surfaces of the membrane cover if it encapsulates the tip, can also be coated with the liner or be otherwise manufactured with low-friction properties.

In many examples, the support tube 35 can also share an external and/or internal lubricious film or coating with the tip 100. The coating can be delivered via dipping, spray, plasma, a profiled mandrel, or any other commonly used technique. Alternately, the membrane cover or jackets can be impregnated with particles having low-friction properties.

In another example, the mouth framework 110 can include an electro-spun or other porous cover that allows for reduced blood flow from the proximal side of the tip-vessel wall seal. A flow reduction between 50% to 99%, more preferably from 60% to 80%, will still direct most of the aspiration flow to the clot while allowing for a small restoring flow portion from the proximal side. This flow can help to reduce the possibility of vessel collapse under excessive aspiration, in locations where vessels have little support from surrounding tissue, or in cases where there are no side branches between a blocked vessel and the expanded tip 100 and a mechanical thrombectomy device or stentriever has not been able to open a portion of the blocked vessel.

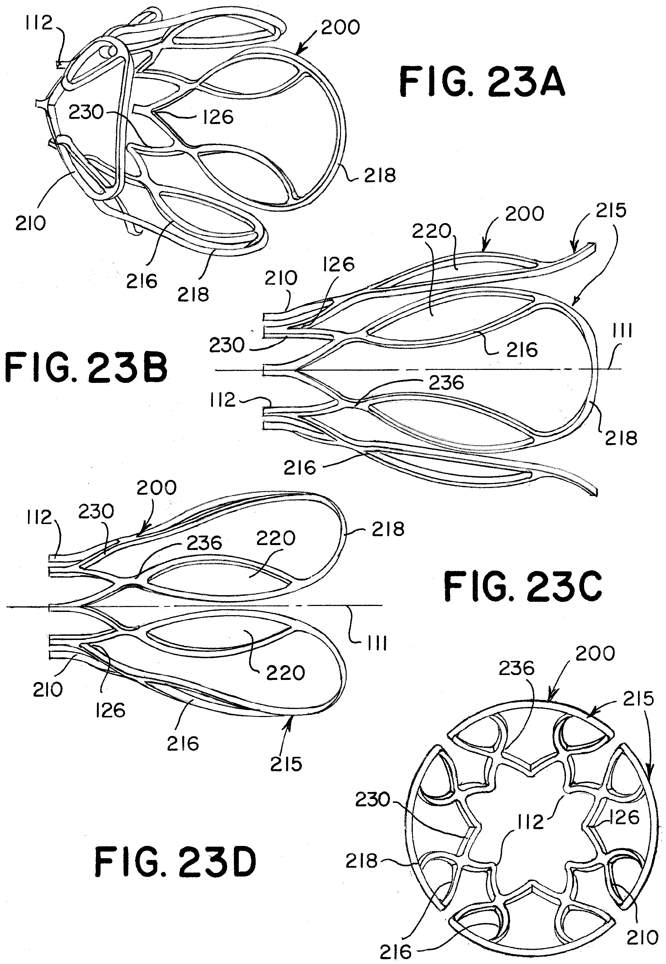

Other mouth support frame examples can have longitudinal supports which are distally independent of one another so they can flex individually. Figs. 23a-d shows one case where an expandable tip 200 has a support frame 210 with a plurality of interconnected struts formed into unconnected petals 215 arranged around the longitudinal axis 111 of the device. The petals 215 can have distal hoop members 218 joining one or more longitudinal arms 216, and the petals can be sized and shaped so that there is no overlapping between them. Alternatively, the petals 215 can be formed in an oversized shape so that they can overlap with one another when held at the desired expanded shape by a membrane cover. Overlapping petals can aid in increasing the radial force capabilities of the frame while sliding relative to each other to share and distribute strains during a procedure.

Similar to other disclosed examples, unconstrained, the petals 215 can expand radially so that a substantially conical surface is formed the combination of arms 216 around the longitudinal axis 111. The struts of the longitudinal arms 216 can branch to form one or more undulations or closed cells 220, allowing the petals 215 to lengthen and shorten independently in response to the forces experienced during navigation through an outer catheter or during a thrombectomy procedure when the tip 200 is deployed and expanded at a target site. Having the longitudinal arms 216 closely aligned with the longitudinal axis 111 of the tip 200 can maintain good column stiffness and pushability characteristics. The petals 215 can also include other members to enhance developed radial force and further support a membrane cover during aspiration.

The support frame 210 can also have proximal support hoops 230 extending distally from support troughs 126 which form connections with a support tube of the catheter, which is not shown for clarity. Connecting struts 236 can form the distal terminating peaks of the proximal support hoops 230 and can link the hoops with the longitudinal arms 216 of the frame.

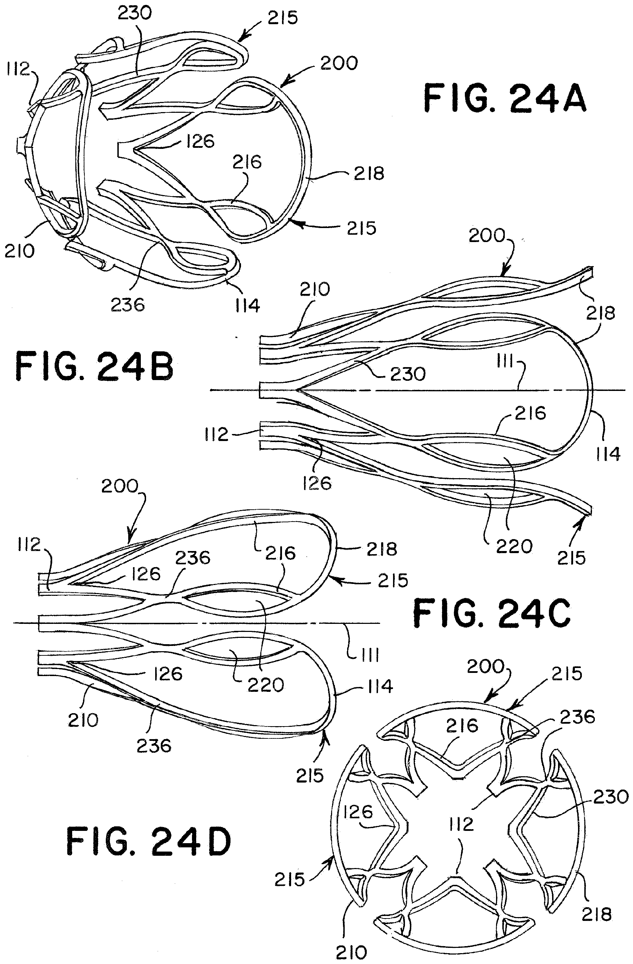

Figs. 24a-d has various views of an alternative support frame 210 for a catheter with distally unconnected petals 215. The struts of the proximal support hoops 230, longitudinal arms 216, and distal hoop members 218 can have a variable or tapering thickness between the proximal end 112 and the distal end 114 of the support frame 210. Struts of the support hoops 230, for example, can be thicker than those of the connecting struts 236, which in turn can have a greater thickness than the struts of the longitudinal arms 216 forming closed cells 220. Similar to local narrowed segments in other examples, this progressive distal thinning of the strut width of the mouth frame 210 can maintain radial force capabilities while keeping the individual petals 215 flexible so they can react independently to lateral loads.

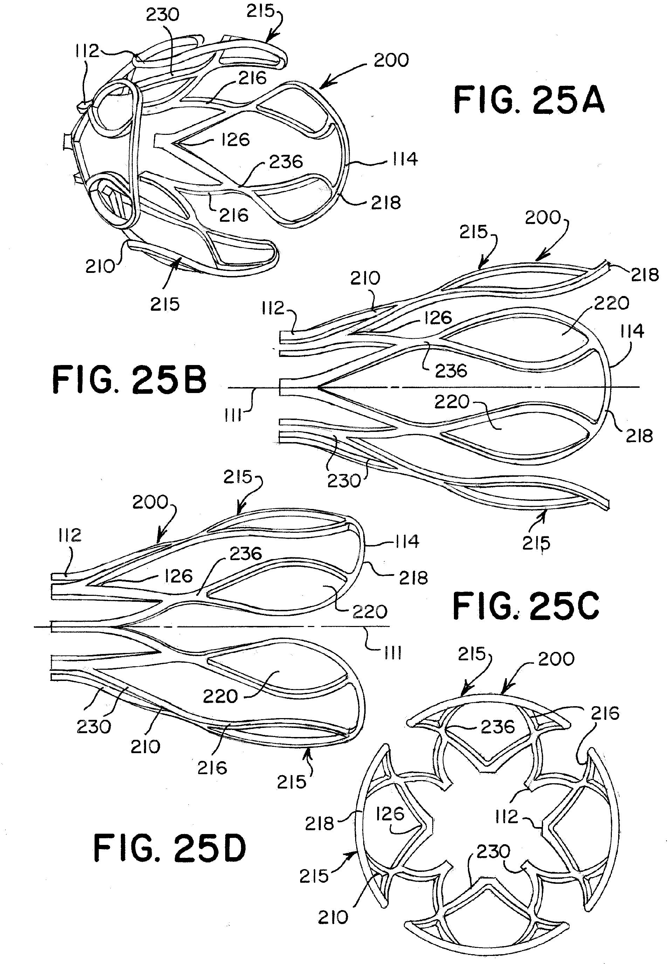

In a similar variant, Figs 25a-d show varied orientations of a support frame 210 for an expandable tip 200 having eight proximal support hoops 230 connecting four distally unconnected petals 215. In Fig. 25b, the support troughs 126 forming the proximal termination for the support hoops can have a staggered axial spacing, and the intersections of struts at the proximal and distal sides of connectors 236 and closed cells 220 can occur at differing angles than those of Fig. 24c to allow for a more equal and balanced strut spacing throughout the frame 210.

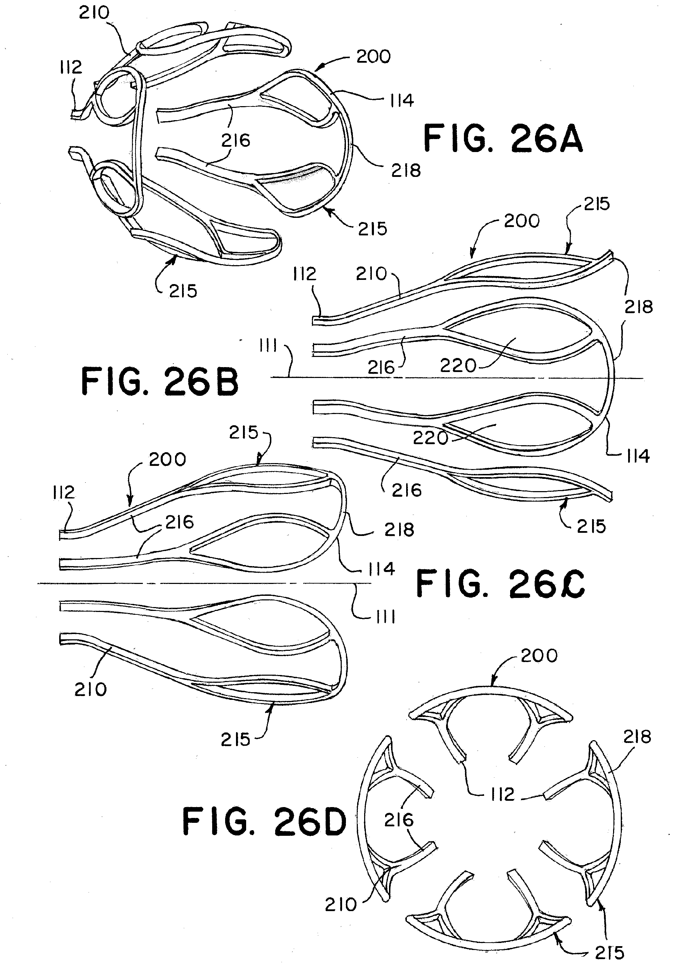

Alternatively, a mouth support frame 210 with distally unconnected petals 215 can have direct connections to the support tube, either to a common axial strut or to the most distal rib or lip (not shown). A support frame with direct connections having eight longitudinal arms 216 joining four distal hoop members 216 to form four unconnected petals 215 is illustrated in Figs. 26a-d. In this example, the direct connections of the longitudinal arms 216 at the proximal end 112 of the frame 210 can replace or supplement the proximal support hoops 230 formed in other designs. Direct connections can allow individual petals to flex and bend more freely about the support frame and with respect to each other when the catheter encounters tortuous advancement paths.

The longitudinal arms 216 can contain one or more closed cells 220 to sustain the necessary radial force while providing additional support for a membrane cover. The spacing of the direct proximal connections and the closed cells can be such that the petals may or may not overlap with each other when held at the desired radial shape of the frame by the cover and can collapse neatly when the tip folded into an outer catheter.

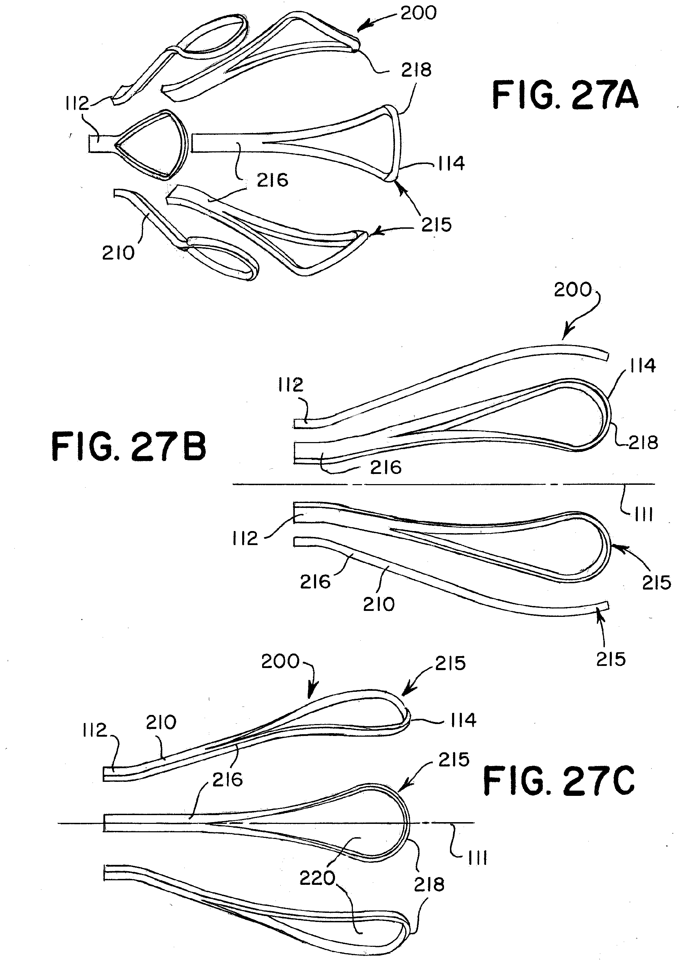

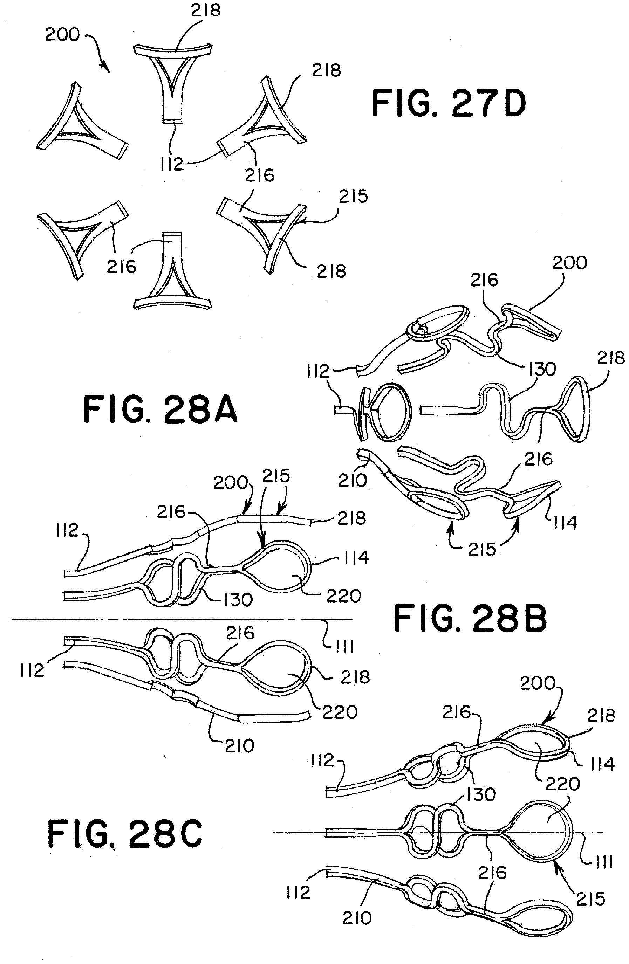

Figs. 27a-d shows an alternative expandable tip 200 where a mouth frame 210 has six distally unconnected petals 215 and six longitudinal arms 216 each extending from a direct connection at the proximal end 112 to a support tube, which is not shown. The longitudinal arms 216 can each include one or more closed cells 220 ending in broadly rounded hoop members 218 at the distal end 114. A portion of the hoop members 218 of the petals 215 can assume a slightly smaller diameter than the peak maximum expanded diameter of the tip 200 profile by curving radially inward. This curve can reduce the likelihood of the distal hoop members 218 pressing into the vessel walls and allow an expanded tip to be briefly advanced through a vessel, when necessary, without causing vessel damage.

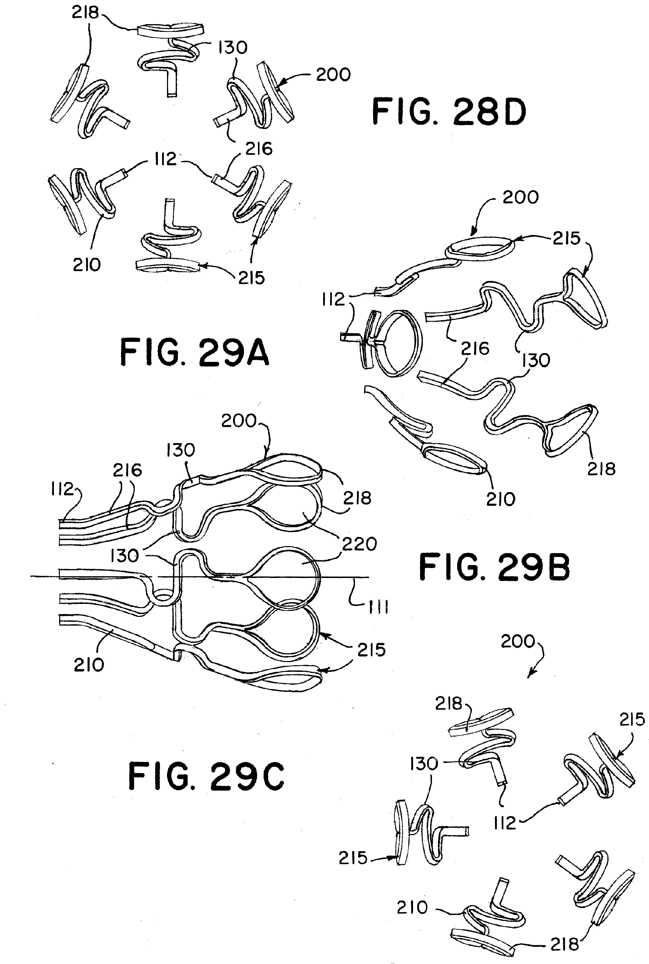

A support frame 210 can also have six longitudinal arms 216 each extending from a direct connection at the proximal end 112 to form six distally unconnected petals 215, as depicted in Figs. 28a-d. When unconstrained, the petals 215 expand radially so the contour of the combination of arms 216 around the longitudinal axis 111 can be substantially conical. One or more of the longitudinal arms 216 can have circumferential undulations 130 or waveform shapes along at least part of their profile. The undulations 130 give the arms a profile which provides more supporting area for a membrane cover while enhancing the lateral flexibility of the frame 210. The supporting area for the cover can be further enhanced by increasing the amplitude of the undulations, reducing their period of oscillation, or both. The undulations 130 can also be staggered or otherwise configured to nest together and fold in a complimentary way when the frame is in the collapsed configuration. Additionally, the longitudinal arms 216 can be capped at the distal end by hoop members 218 to form loops or closed cells 220. The distal most end of the closed cells 220 of the arms 216 can taper inwardly from a maximum expanded diameter of the frame, as illustrated in Fig. 28b and Fig. 28d to give the frame 210 a more atraumatic profile.

As observed from Figs. 29a-c, an alternate expandable mouth support frame 210 can have five unconnected petals 215 equally spaced around a longitudinal axis 111. The longitudinal arm 216 making up each petal can be connected independently to a support tube. The struts of the longitudinal arms 216 can have a first thickness near the proximal end 112 different than a second thickness of the struts at a more distal region of the arms to combine good pushability with distal flexibility. Similar to other examples, the arms 216 can have undulations 130 to allow the arms to bend and flex independently about their own axes and can have distal closed cells 220 providing greater support for a membrane cover.

It can be appreciated that fewer unconnected petals 215 in Figs. 29a-c as compared to the six or more of the designs of other examples can give an atraumatic curve to the mouth of the tip and increased bending flexibility for a given strut thickness and width. To maintain adequate support for a membrane cover, a design with fewer petals can incorporate additional undulations 130 and/or closed cells 220 sufficient provide decent radial force and to prevent the cover from collapsing under the suction force of aspiration during a procedure.

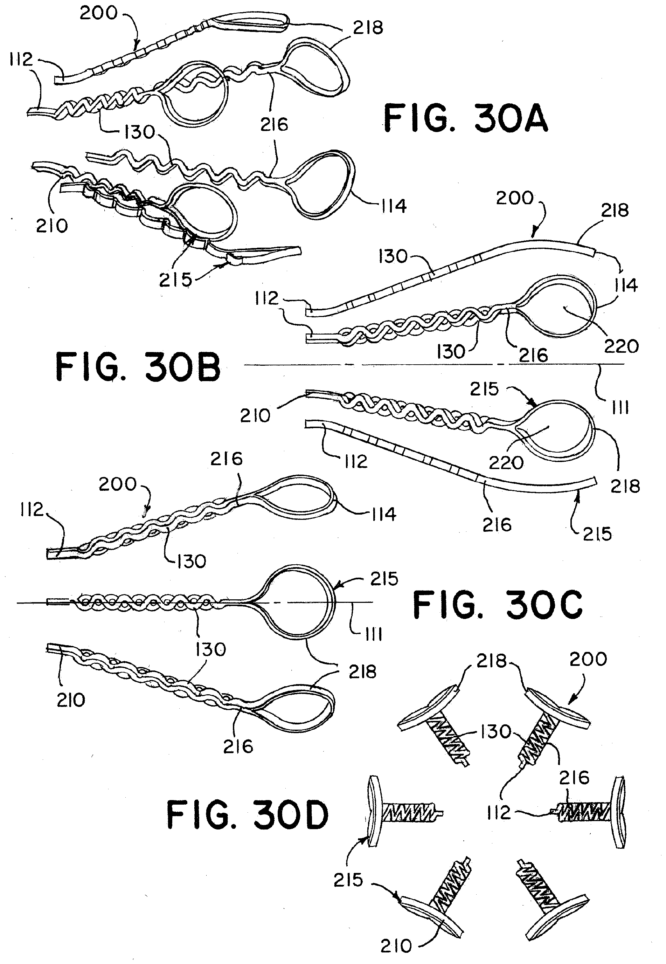

Several views of a support frame 210 having a plurality of undulations or waveforms 130 along the length of the longitudinal arms 216 and with an array of closed cells 220 around the longitudinal axis 111 at the distal end 114 is considered in Figs. 30a-d. The undulations 130 can be circumferential and have a smooth periodic oscillation, or the arms and oscillations can twist in a helical direction with respect to the axis 111. The additional length of undulations in an arm 216 allow each petal 215 to better torque and bend about the axis longitudinal of the arm independently of the other petals to provide a device that will track more easily through an outer catheter when the frame is in a collapsed state. A helical twist in the arms 216 can further aid the petals 215 in torqueing through tortuous vessel anatomies.

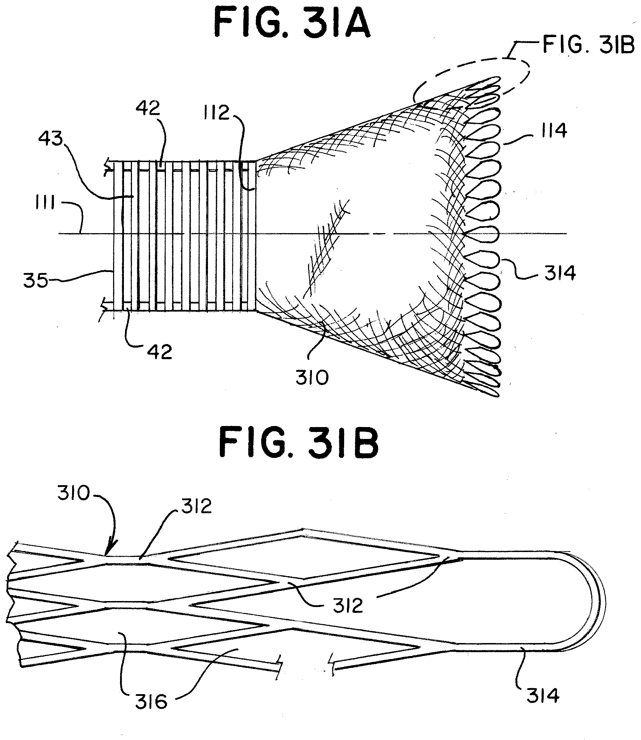

In another alternative construction, an expandable mouth tip 300 of a catheter can have a radial array of struts or strands organized into a closed cell mesh 310, as illustrated in Figs. 31a-b. The mesh can have a proximal end 112, a distal end 114, and form a substantially conical or funnel-like shape around a longitudinal axis 111 when unconstrained and allowed to expand upon exiting an outer catheter. The mesh array can be made of wire or cut from a shape memory alloy such that the mouth can be heat set to self-expand from a collapsed delivery configuration to an enlarged deployed configuration. The mesh 310 can be adhered or otherwise connected at the proximal end 112 to a support tube 35 of the catheter. The mesh pattern 310 can be manufactured so as to have a single circumferential joint for attaching the proximal end, or when the support tube 35 has a structure of ribs 43 and spines 42, the individual strands of the mesh can be bonded to the most distal rib of the tube. A flexible polymeric membrane 50 (such as that seen in Fig. 22) can cover some or all of the closed cell mesh 310 of the catheter tip 300.

In another example, the support tube 35 can have a metal and/or polymer strand construction formed into a patterned mesh or coiled structure. The structure can form a radial array as a continuous tubular catheter body and in some cases can even be integral with the expandable tip 300. In this case the stiffness transition between the support tube 35 and tip 300 is minimized in order to approximate a singular supporting piece and better distribute strain. The tube can be coated or encapsulated with a cover or membrane to provide smooth surfaces for trackability within an outer catheter and the internal passage of ancillary devices.

Fig. 31b shows a closer view demonstrating a possible repeating pattern of the closed cell mesh 310 of the expandable tip 300. When cut from a flat pattern, the radial profile of the formed mesh pattern 310 can be straight as shown, with a constant angle with respect to the longitudinal axis 111. In other examples, the profile can be a more broadly flared concave arc to form a more atraumatic and rounded outer surface for contact with the walls of a vessel.

The closed cell mesh array 310 making up the mouth framework can be a continuous polygonal pattern as shown such as triangular or quadrilateral cells which are interlocked through the sharing of the vertices of the adjacent cells. In one case, the array 310 can have an elongated quadrilateral pattern as seen in Fig. 31b where individual closed cells are formed where local array peaks 312 mark the shared vertices. The pattern can repeat in an axial and radial fashion and the distalmost array peaks 312 of adjacent cells can be joined by curved distal hoops 314 or crowns to mark the perimeter of the expandable mouth. Similarly, the peaks of the distal hoops can be joined together by a single fully circumferential hoop or crown to prevent individual distal hoops from catching or snagging on branched vessels or similar features.

The cells formed by the shared array peaks 312 define the open pores 316 of the closed cell mesh 310 structure. The pores 316 of the mesh can be sized so as to tailor the filtration properties of the expandable tip 300. For example, large pores can give the tip enhanced flexibility yielding deliverability advantages while incorporating an outer membrane cover or jacket (not shown) to block or limit blood flow from regions proximal to the tip when deployed in the expanded configuration at a target site.

Alternately, the pores 316 can be micro-sized to form a mesh array 310 which is dense enough to impede flow sufficiently to where an outer jacket or a membrane cover is not necessary. In this case, the outer cover of the support tube 35 can terminate close to or in a region just distal of the proximal end 112 of the expandable tip 300 where the diameter of the tip begins to expand in the deployed configuration. By not requiring an outer membrane cover, the expandable tip 300 can track more easily through an outer catheter and the required advancement forces can be limited. As a result, although one may still be applied, a lubricious or low-friction coating may not be required.

There are a wide variety of minimally invasive stent patterns, meshes, or screens found in commercially available products with a range of capabilities and applications. It can be appreciated that the closed cell mesh 310 of the expandable tip 300 can utilize any expanding stent pattern known in the art of stent patents and products and that the pore 316 sizes need not be restricted to those disclosed herein.

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near to or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. Similarly, it is also to be understood that the mention of one or more components in a device or system does not preclude the presence of additional components or intervening components between those components expressly identified. The mention of one or more steps of a method does not preclude the presence of additional method steps or intervening method steps between those steps expressly identified. For clarity and conciseness, not all possible combinations have been listed, and such modifications are often apparent to those of skill in the art. The scope of the invention is defined by the claims.

## Claims

1. An expandable mouth (100) for a clot retrieval catheter, the expandable mouth comprising:
a proximal end;
a distal end;
a longitudinal axis; and
a self-expanding mouth framework (110) comprising a plurality of interconnected struts, a collapsed delivery configuration and expanded deployed configuration, and a polymeric membrane cover, the struts of the mouth framework comprising:
one or more support arms (116) extending longitudinally between the proximal end and the distal end and forming a substantially conical surface about the longitudinal axis in the deployed configuration, and
one or more distal hoops (118) forming a circumferential perimeter of a mouth opening of the clot retrieval catheter; and
**characterised in that** the one or more support arms comprise at least one circumferential undulation (130).

2. The expandable mouth (100) of claim 1, further comprising a support tube (35) connected proximally to the mouth framework (110), the support tube comprising a plurality of ribs and one or more axial spines (42).

3. The expandable mouth (100) of claim 1, wherein the mouth framework (110) further comprises one or more narrowed segments (124).

4. The expandable mouth (100) of claim 2, wherein at least one axial spine (42) of the support tube (35) is axially aligned with a support arm (116).

5. The expandable mouth (100) of claim 1, wherein in the deployed state the mouth framework (110) is tapered such that a proximal portion of the mouth framework has a first radial size and a distal portion of the mouth framework has a second radial size larger than the first maximum radial size.

6. The expandable mouth (100) of claim 5, wherein the second maximum radial size is at least 2.5 mm in diameter and sized to be larger than the inner diameter of a target blood vessel.

7. The expandable mouth (100) of claim 1, wherein at least a portion of the distal hoop or hoops (118) extends radially inward in a distal direction from the maximum radial size of the mouth framework (110).

8. The expandable mouth (100) of claim 1, wherein at least a portion of the struts of the support arms (116) have a width different than the width of at least a portion of the width of the struts of the distal hoops (118).

9. The expandable mouth (100) of claim 1, wherein the struts of at least one of the one or more support arms (116) terminate at a support trough (126) and a hoop trough (121) to form a closed cell.

10. The expandable mouth (100) of claim 2, wherein the struts of at least one of the one or more support arms (116) terminate at the support tube (35) and a hoop trough (121) to form a closed cell.

11. The expandable mouth (100) of claim 1, further comprising:
an angle formed between the support arms (116) and the longitudinal axis, the angle having a range from approximately 10 degrees to approximately 45 degrees.

12. The expandable mouth (100) of claim 1, further comprising:
an angle formed between the support arms (116) and the longitudinal axis is approximately 30 degrees.

## Patentansprüche

1. Expandierbarer Mund (100) für einen Katheter zum Entfernen von Blutgerinnseln, wobei der expandierbare Mund umfasst:
ein proximales Ende;
ein distales Ende;
eine Längsachse; und
einen selbst-expandierenden Mundrahmen (110), der eine Vielzahl von miteinander verbundenen Streben, eine zusammengefaltete Zuführauslegung und eine expandierte ausgebrachte Auslegung und eine Polymermembranabdeckung umfasst, wobei die Streben des Mundrahmens umfassen:
einen oder mehrere Stützarme (116), die sich in Längsrichtung zwischen dem proximalen Ende und dem distalen Ende erstrecken und in der ausgebrachten Auslegung eine im Wesentlichen konische Fläche um die Längsachse bilden; und
einen oder mehrere distale Ringe (118), die einen Umfang einer Mundöffnung des Katheters zum Entfernen von Blutgerinnseln bilden; und
**dadurch gekennzeichnet, dass** der eine oder die mehreren Stützarme mindestens eine umlaufende Wellung (130) umfassen.

2. Expandierender Mund (100) nach Anspruch 1, ferner umfassend eine Stützröhre (35), die proximal mit dem Mundrahmen (110) verbunden ist, wobei die Stützröhre eine Vielzahl von Rippen und einen oder mehrere axiale Rücken (42) umfasst.

3. Expandierbarer Mund (100) nach Anspruch 1, wobei der Mundrahmen (110) ferner ein oder mehrere verschmälerte Segmente (124) umfasst.

4. Expandierbarer Mund (100) nach Anspruch 2, wobei mindestens ein axialer Rücken (42) der Stützröhre (35) axial auf einen Stützarm (116) ausgerichtet ist.

5. Expandierbarer Mund (100) nach Anspruch 1, wobei der Mundrahmen (110) in dem ausgebrachten Zustand konisch ist, so dass ein proximaler Abschnitt des Mundrahmens eine erste radiale Größe aufweist und ein distaler Abschnitt des Mundrahmens eine zweite radiale Größe aufweist, die größer ist als die erste maximale radiale Größe.

6. Expandierbarer Mund (100) nach Anspruch 5, wobei die zweite maximale radiale Größe mindestens 2,5 mm im Durchmesser beträgt und dazu bemessen ist, größer zu sein als der Innendurchmesser eines Zielblutgefäßes.

7. Expandierbarer Mund (100) nach Anspruch 1, wobei sich mindestens ein Abschnitt des distalen Ringes oder der distalen Ringe (118) von der maximalen radialen Größe des Mundrahmens (110) in eine distale Richtung radial nach innen erstreckt.

8. Expandierbarer Mund (100) nach Anspruch 1, wobei mindestens ein Abschnitt der Streben der Stützarme (116) eine Breite aufweist, die sich von der Breite von mindestens einem Abschnitt der Breite der Streben der distalen Ringe (118) unterscheidet.

9. Expandierbarer Mund (100) nach Anspruch 1, wobei die Streben von mindestens einem des einen oder der mehreren Stützarme (116) in eine Stützmulde (126) und eine Ringmulde (121) münden, um eine geschlossene Zelle zu bilden.

10. Expandierbarer Mund (100) nach Anspruch 2, wobei die Streben von mindestens einem des einen oder der mehreren Stützarme (116) in die Stützröhre (35) und eine Ringmulde (121) münden, um eine geschlossene Zelle zu bilden.

11. Expandierbarer Mund (100) nach Anspruch 1, ferner umfassend:
einen Winkel, der zwischen den Stützarmen (116) und der Längsachse gebildet ist, wobei der Winkel einen Bereich von ungefähr 10 Grad bis ungefähr 45 Grad aufweist.

12. Expandierbarer Mund (100) nach Anspruch 1, ferner umfassend:
einen zwischen den Stützarmen (116) und der Längsachse gebildeten Winkel von ungefähr 30 Grad.

## Revendications

1. Bouche extensible (100) pour un cathéter de récupération de caillots, la bouche extensible comprenant :
une extrémité proximale ;
une extrémité distale ;
un axe longitudinal ; et
un cadre de bouche auto-extensible (110) comprenant une pluralité d'entretoises interconnectées, une configuration de mise en place repliée et une configuration déployée étendue, et une membrane de couverture polymère, les entretoises du cadre de bouche comprenant :
un ou plusieurs bras de support (116) s'étendant longitudinalement entre l'extrémité proximale et l'extrémité distale et formant une surface sensiblement conique autour de l'axe longitudinal dans la configuration déployée, et
un ou plusieurs arceaux distaux (118) formant un périmètre circonférentiel de l'ouverture de l'embouchure du cathéter de prélèvement de caillots ; et
**caractérisé en ce que** le ou les bras de support comportent au moins une ondulation circonférentielle (130).

2. Bouche extensible (100) selon la revendication 1, comprenant en outre un tube de support (35) relié proximalement au cadre de bouche (110), le tube de support comprenant une pluralité de nervures et une ou plusieurs épines axiales (42).

3. Bouche extensible (100) selon la revendication 1, le cadre de bouche (110) comprenant en outre un ou plusieurs segments rétrécis (124).

4. Bouche extensible (100) selon la revendication 2, au moins une épine axiale (42) du tube de support (35) étant alignée axialement avec un bras de support (116).

5. Bouche extensible (100) selon la revendication 1, à l'état déployé, le cadre de bouche (110) étant effilé de sorte qu'une partie proximale du cadre de bouche a une première taille radiale et une partie distale du cadre de bouche a une seconde taille radiale plus grande que la première taille radiale maximale.

6. Bouche extensible (100) selon la revendication 5, la seconde taille radiale maximale ayant un diamètre d'au moins 2,5 mm et étant dimensionnée pour être plus grande que le diamètre interne d'un vaisseau sanguin cible.

7. Bouche extensible (100) selon la revendication 1, au moins une partie du ou des arceaux distaux (118) s'étendant radialement vers l'intérieur dans une direction distale à partir de la taille radiale maximale du cadre de bouche (110).

8. Bouche extensible (100) selon la revendication 1, au moins une partie des entretoises des bras de support (116) ayant une largeur différente de la largeur d'au moins une partie de la largeur des entretoises des arceaux distaux (118).

9. Bouche extensible (100) selon la revendication 1, les entretoises d'au moins un du ou des bras de support (116) se terminant au niveau d'une cuvette de support (126) et d'une cuvette de cerceau (121) pour former une cellule fermée.

10. Bouche extensible (100) selon la revendication 2, les entretoises d'au moins un du ou des bras de support (116) se terminant au niveau du tube de support (35) et d'une cuvette de cerceau (121) pour former une cellule fermée.

11. Bouche extensible (100) selon la revendication 1, comprenant en outre :
un angle formé entre les bras de support (116) et l'axe longitudinal, l'angle étant compris entre environ 10 degrés et environ 45 degrés.

12. Bouche extensible (100) selon la revendication 1, comprenant en outre :
un angle formé entre les bras de support (116) et l'axe longitudinal d'environ 30 degrés.
